(19) Europäisches Patentamt
European Patent Office
Office européen des brevets

(11) **EP 1 641 815 B1**

(12) **FASCICULE DE BREVET EUROPEEN**

(45) Date de publication et mention
de la délivrance du brevet:
**27.05.2015 Bulletin 2015/22**

(51) Int Cl.:
*C07K 14/14* (2006.01)     *C07K 14/47* (2006.01)
*C12N 15/62* (2006.01)     *C12N 5/10* (2006.01)
*C07K 16/18* (2006.01)

(21) Numéro de dépôt: **04767541.8**

(22) Date de dépôt: **01.07.2004**

(86) Numéro de dépôt international:
**PCT/FR2004/001698**

(87) Numéro de publication internationale:
**WO 2005/012336 (10.02.2005 Gazette 2005/06)**

(54) **PEPTIDES CHELATANT L URANIUM ET LEURS APPLICATIONS**

URAN-CHELATISIERENDE PEPTIDE UND DEREN VERWENDUNG

URANIUM-CHELATING PEPTIDES AND USES THEREOF

(84) Etats contractants désignés:
**AT BE BG CH CY CZ DE DK EE ES FI FR GB GR HU IE IT LI LU MC NL PL PT RO SE SI SK TR**

(30) Priorité: **04.07.2003  FR 0308211**

(43) Date de publication de la demande:
**05.04.2006  Bulletin 2006/14**

(73) Titulaire: **Commissariat à l'Énergie Atomique et aux Énergies Alternatives
75015 Paris (FR)**

(72) Inventeurs:
  • **VITA, Claudio**
    **91190 Gif-sur-Yvette (FR)**
  • **LE CLAINCHE, Loïc**
    **75013 Paris (FR)**
  • **MONJARDET, Véronique**
    **75014 Paris (FR)**

(74) Mandataire: **Leblois-Préhaud, Hélène Marthe Georgette et al
Cabinet Orès
36, rue de St Pétersbourg
75008 Paris (FR)**

(56) Documents cités:
  • **LE CLAINCHE LOIC ET AL: "Engineering new metal specificity in EF-hand peptides." JBIC JOURNAL OF BIOLOGICAL INORGANIC CHEMISTRY, vol. 8, no. 3, 20 novembre 2002 (2002-11-20), pages 334-340, XP002313279 ISSN: 0949-8257**

  • **FISCHER R ET AL: "MULTIPLE DEVERGENT MRNAS CODE FOR A SINGLE HUMAN CALMODULIN" JOURNAL OF BIOLOGICAL CHEMISTRY, AMERICAN SOCIETY OF BIOLOGICAL CHEMISTS, BALTIMORE, MD, US, vol. 263, no. 32, 15 novembre 1988 (1988-11-15), pages 17055-17062, XP000073231 ISSN: 0021-9258 -& DATABASE SWISSPROT [Online] 15 mars 2004 (2004-03-15), "Calmodulin" XP002277766 extrait de SWISSPROT Database accession no. P02593**
  • **PROCYSHYN RIC M ET AL: "A structure/activity study of calcium affinity and selectivity using a synthetic peptide model of the helix-loop-helix calcium-binding motif" JOURNAL OF BIOLOGICAL CHEMISTRY, vol. 269, no. 3, 1994, pages 1641-1647, XP002318116 ISSN: 0021-9258**
  • **MARSDEN B J ET AL: "CALCIUM BINDING PROTEINS ELUCIDATING THE CONTRIBUTIONS TO CALCIUM AFFINITY FROM AN ANALYSIS OF SPECIES VARIANTS AND PEPTIDE FRAGMENTS" BIOCHEMISTRY AND CELL BIOLOGY, vol. 68, no. 3, 1990, pages 587-601, XP009042755 ISSN: 0829-8211**
  • **BENDER P K ET AL: "THE ABUNDANCE OF CALMODULIN MESSENGER RNA IS REGULATED IN PHOSPHORYLASE-KINASE-DEFICIENT SKELETAL MUSCLE" JOURNAL OF BIOLOGICAL CHEMISTRY, vol. 263, no. 20, 1988, pages 9733-9737, XP002318462 ISSN: 0021-9258**

- RHYNER J A ET AL: "STRUCTURE OF THE HUMAN CALM1 CALMODULIN GENE AND IDENTIFICATION OF TWO CALM1-RELATED PSEUDOGENES CALM1P1 AND CALM1P2" EUROPEAN JOURNAL OF BIOCHEMISTRY, BERLIN, DE, vol. 225, 1994, pages 71-82, XP002949255 ISSN: 0014-2956 -& DATABASE SWISSPROT [Online] 1 mai 2005 (2005-05-01), "Calmodulin (CaM)" XP002318463 extrait de SWISSPROT Database accession no. P62158
- BUCHTA R ET AL: "PEPTIDES RELATED TO THE CALCIUM BINDING DOMAINS II AND III OF CALMODULIN SYNTHESIS AND CALMODULIN-LIKE FEATURES" INTERNATIONAL JOURNAL OF PEPTIDE AND PROTEIN RESEARCH, vol. 28, no. 3, 1986, pages 289-297, XP009042763 ISSN: 0367-8377
- BABU Y S ET AL: "STRUCTURE OF CALMODULIN REFINED AT 2.2 A RESOLUTION" JOURNAL OF MOLECULAR BIOLOGY, vol. 204, no. 1, 1988, pages 191-204, XP009029518 ISSN: 0022-2836
- WILSON M A ET AL: "The 1.0 A crystal structure of Ca<2+>-bound calmodulin: an analysis of disorder and implications for functionally relevant plasticity" JOURNAL OF MOLECULAR BIOLOGY, LONDON, GB, vol. 301, no. 5, 1 septembre 2000 (2000-09-01), pages 1237-1256, XP004461694 ISSN: 0022-2836
- REID R E: "SYNTHETIC FRAGMENTS OF CALMODULIN CALCIUM-BINDING SITE III A TEST OF THE ACID PAIR HYPOTHESIS" JOURNAL OF BIOLOGICAL CHEMISTRY, vol. 265, no. 11, 1990, pages 5971-5976, XP002318118 ISSN: 0021-9258
- "The EF-hand Calcium-binding Proteins Data Library"[Online] XP002318119 Extrait de l'Internet: URL:http://structbio.vanderbilt.edu/cabp_d atabase/> [extrait le 2005-01-17]
- MCCORMACK ELIZABETH ET AL: "Calmodulins and related potential calcium sensors of Arabidopsis." NEW PHYTOLOGIST, vol. 159, no. 3, septembre 2003 (2003-09), pages 585-598, XP002318120 ISSN: 0028-646X
- BERTINI IVANO ET AL: "Tuning the affinity for lanthanides of calcium binding proteins." BIOCHEMISTRY, vol. 42, no. 26, 8 juillet 2003 (2003-07-08), pages 8011-8021, XP002318121 ISSN: 0006-2960
- BHATTACHARYA SHIBANI ET AL: "Target selectivity in EF-hand calcium binding proteins." BIOCHIMICA ET BIOPHYSICA ACTA. 6 DEC 2004, vol. 1742, no. 1-3, 15 septembre 2004 (2004-09-15), pages 69-79, XP002318122 ISSN: 0006-3002
- FINN B E ET AL: "The evolving model of calmodulin structure,function and activation" STRUCTURE, CURRENT BIOLOGY LTD., PHILADELPHIA, PA, US, vol. 3, no. 1, janvier 1995 (1995-01), pages 7-11, XP004587826 ISSN: 0969-2126
- NELSON MELANIE R ET AL: "Structures of EF-hand Ca2+-binding proteins: Diversity in the organization, packing and response to Ca2+ binding" BIOMETALS, vol. 11, no. 4, décembre 1998 (1998-12), pages 297-318, XP002318123 ISSN: 0966-0844
- REID RONALD E ET AL: "Engineering magnesium selectivity in the helix-loop-helix calcium-binding motif" ARCHIVES OF BIOCHEMISTRY AND BIOPHYSICS, vol. 323, no. 1, 1995, pages 115-119, XP002318479 ISSN: 0003-9861

**Description**

La présente invention est relative à des peptides chélatant l'uranium ainsi qu'à leurs applications pour la décontamination des sols et des eaux, ainsi que pour la détection et le traitement des personnes contaminées par l'uranium.

L'uranium est normalement présent dans l'environnement en concentration très faible, sous la forme d'isotopes 238 (99,27%) et 235 (0,72%) qui se décomposent par émission de particules alpha de faible toxicité radiologique ; la forme uranyle ($UO_2^{2+}$) représente la forme la plus commune de l'uranium dans une atmosphère oxygénée.

Toutefois, dans des sites particuliers, comme près des mines de minéraux d'uranium, des sites de stockage (contrôle nucléaire ou stockage de munitions à l'uranium appauvri) ou bien dans le cas d'accident nucléaire, la concentration de ce métal peut être beaucoup plus élevée et représente un danger pour l'homme, du fait de son accumulation dans les reins et dans les os : toxicité pour le tissu rénal et développement de cancers du tissu osseux.

La décontamination des sites et des personnes contaminés nécessite de disposer, d'une part de moyens de neutraliser la toxicité de l'uranium dans l'environnement et dans l'organisme des individus contaminés, et d'autre part de réactifs de détection, efficaces et spécifiques pour ce métal. Toutefois, il n'existe pas actuellement de moyens efficaces de détection et de décontamination de l'uranium, notamment du fait de l'absence de ligands spécifiques de l'uranium, capables de détecter l'uranium (senseur) et de chélater ce métal toxique qui pourrait être présent dans un environnement et/ou dans un milieu biologique contaminé, afin de réaliser sa décontamination.

Le traitement actuel de décontamination des sols est effectué principalement par l'excavation, la récolte et le stockage dans des sites appropriés ou par l'extraction de l'uranium à l'aide d'agents chélatants ; ces traitements physicochimiques sont chers, peu ou pas spécifiques et peu adaptés au traitement de surfaces contaminées très étendues et présentent en outre un risque de contamination élevé pour les opérateurs, du fait d'une exposition répétée à l'uranium. Alternativement, il a été proposé d'utiliser des organismes vivants (microorganismes ou végétaux supérieurs) pour décontaminer les sols et les eaux contaminés par l'uranium. Cette méthode repose sur l'absorption des métaux et donc sur leur séquestration par ces organismes. Par exemple, des végétaux sont capables d'absorber les métaux toxiques par les racines et de les accumuler dans les feuilles, qui sont ensuite récoltées et stockées dans des sites appropriés ; les organismes actuellement disponibles ne permettent pas une décontamination efficace des sols et des eaux contaminés du fait de leurs faibles capacités d'extraction, de tolérance et d'accumulation de concentrations élevées de métaux toxiques.

La détection de l'uranium chez les personnes susceptibles d'avoir été contaminées par l'uranium est réalisée *ex situ* par spectrométrie de masse à plasma (ICP-MS) ; cette technique est lourde à mettre en oeuvre et onéreuse.

Le traitement des personnes contaminées par l'uranium est effectué par administration d'agents chélatants qui lient l'uranium, favorisant ainsi son excrétion et réduisant par conséquent son dépôt dans les reins et les os. Parmi les principaux agents chélatants de l'uranium, on peut citer : l'acide diéthylène triamine pentacétique (DTPA), l'acide 5-aminosalicylique (5-AS), l'acide gallique, le sulfocathécole, le carboxycathécole et l'hydroxypyridinone ; ces agents chélatants présentent l'inconvénient de ne pas être spécifiques de l'uranium.

Différentes approches ont été développées pour détecter spécifiquement certains métaux, notamment en milieu aqueux ou dans des échantillons biologiques :

- des senseurs chimiques fluorescents (Tsien, 1993 : Fluorescent chemosensors for ion and molecule recognition, pages 130-146, Czarnik AW (ed), American Chemical Society, Washington DC*) ;* ces senseurs fluorescents sont spécifiques du sodium, du potassium, du calcium et du magnésium ; en revanche, aucun senseur chimique spécifique de l'uranium n'a été décrit.

- des senseurs peptidiques fluorescents (biosenséurs) constitués par un peptide d'environ 26 acides aminés dérivé d'un domaine en doigt à zinc ou *Zinc finger domain,* marqué par au moins un groupement fluorescent (Walkup et al., 1996, J. Am. Soc., 119, 3443-3450 ; Godwin et al., 1998, J. Am. Soc., 118, 6514-6515 ; Walkup et al., 1997, J. Am. Soc., 119, 3443-3540). En présence d'ions zinc, ces biosenseurs peptidiques se structurent autour du métal et exposent le groupement fluorescent à des changements d'environnement qui se traduisent par une variation d'émission de fluorescence qui est fonction de la concentration du métal. Alternativement, lorsque les peptides sont conjugués à deux groupements fluorescents appropriés, la liaison du zinc au peptide entraîne une modification de conformation favorable à un transfert efficace d'énergie entre les deux fluorophores (*Fluorescence Resonance Energy Transfer* ou FRET), résultant en l'émission d'un signal fluorescent proportionnel à la concentration du métal (Walkup et al., 1996, précité). Du fait de la structure du domaine en « doigt à zinc » qui est adaptée à la chélation d'ions à géométrie tétraédrique, comme le zinc, ces biosenseurs ne permettent pas in chélation de l'uranium, qui sous sa forme uranyle ($U0_2{}^{2+}$) la plus courante en milieu oxygéné, présente une géométrie bipyramidale à base pentagonale ou hexagonale avec un nombre de coordination de 7-8 (uranium VI).

- des senseurs protéiques fluorescents, dénommés « caméléons », constitués par une protéine de fusion comprenant successivement de son extrémité $NH_2$ vers son extrémité COOH : un mutant bleu ou cyan (EBFP ou ECFP, donneur de fluorescence) de la protéine fluorescente GFP dérivée de la méduse *Aequorea victoria,* la calmoduline (CaM) comprenant les domaines N- et C- terminaux et les sites I et II de fixation de l'ion calcium, un peptide de 26 résidus se liant à la calmoduline et dérivé du domaine liant la calmoduline d'une kinase de la chaîne légère de la myosine (MLCK), et un autre mutant vert ou jaune de la même protéine fluorescente (EGFP ou EYFP, accepteur de fluorescence), (Miyawaki et al., Nature, 1997, 388, 882-887). La liaison du calcium à la calmoduline provoque un changement conformationnel dans la protéine de fusion, qui forme un nouveau site auquel se fixe le peptide, et qui produit une association entre les deux protéines fluorescentes et un positionnement dans l'espace favorable à un transfert efficace d'énergie du donneur (EBFP ou ECFP) vers l'accepteur de fluorescence (EGFP ou EYFP), produisant ainsi une augmentation de la fluorescence émise par l'accepteur de fluorescence (EGFP ou EYFP). D'autres indicateurs fluorescents « caméléons » plus sensibles et spécifiques pour une gamme de concentrations de calcium plus étendue ont également été obtenus (Truong et al., Nature Struct. Biol., 2001, 8, 1069-1073). Ce système d'indicateur fluorescent qui repose sur les variations de conformation induites par la liaison du calcium au complexe calmoduline-MLCKp est spécifique du calcium et ne permet donc pas de détecter d'autres ions métalliques, comme par exemple l'uranyle.

- des ligands peptidiques sélectifs de métaux lourds, dérivés de motifs hélice-boucle-hélice (Borin et al., Biopolymer, 1989, 28, 353-369 ; Dadlez et al., FEBS Lett., 1991, 282, 143, 146 ; Marsden et al., Biochem. Cell. Biol., 1990, 68, 587-601 ; Shaw et al., Science, 1990, 249, 280-283 ; Reid et al., Arch. Biochem. Biophys., 1995, 323, 115-119; Procyshyn et al., J. Biol. Chem., 1994, 269, 1641-1647) ; ces peptides présentent une faible structuration de leurs hélices en milieux aqueux, ainsi que de faibles affinités pour les métaux divalents (Kd de l'ordre du millimolaire).

- des bactéries contenant un promoteur pouvant traduire la présence d'un métal toxique en un signal lumineux (Bechor et al. Biotechnol., 2002, 94, 125-132; Lee et al., Biosen. Bioelectron., 2003, 18, 571-577) ; dans ces systèmes, le toxique agit comme facteur de stress cellulaire et induit ainsi une expression altérée d'une protéine bioluminescente, qui représente le signal détecté ; ces systèmes ne sont donc pas spécifiques de l'uranium et des métaux toxiques en général.

Des peptides correspondants au site I de la calmoduline ont été synthétisés. La mutation du résidu d'acide glutamique de la boucle de coordination du calcium (E31D) abolit l'affinité pour le calcium mais préserve l'affinité pour les lanthanides montrant que la sélectivité pour les métaux peut être modulée par des mutations spécifiques (Le Clainche et al., J. Biol. Inorg. Chem., 2003, 8, 334-340).

Il ressort de ce qui précède qu'il n'existe pas actuellement de ligands spécifiques de l'uranium, capables de détecter (senseur) et de chélater ce métal toxique qui pourrait être présent dans un environnement et/ou dans un milieu biologique contaminé, afin de réaliser sa décontamination. La chélation spécifique de l'uranium dans l'environnement (sol, eau, ..) et dans l'organisme vivant est néanmoins difficile à réaliser, du fait de la présence de fort excès d'autres métaux, comme les métaux alcalino-terreux ou les lanthanides qui sont compétiteurs pour la fixation de l'uranium.

En conséquence, les Inventeurs se sont donnés pour but de pourvoir à un agent capable de chélater spécifiquement l'uranium (VI), sous la forme d'uranyle ($UO_2^{2+}$).

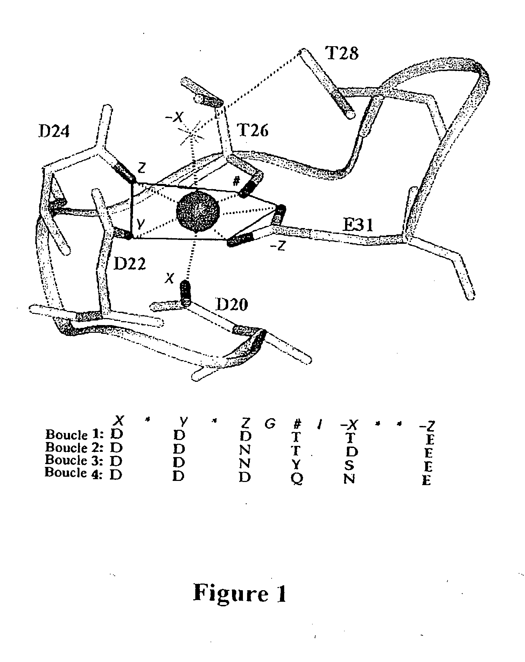

De masse 16,7 kDa, la calmoduline est ubiquitaire dans les cellules eucaryotes et joue un rôle important dans la traduction du signal entre différents compartiments cellulaires médié par des variations de la concentration en calcium. En réponse à une augmentation de la concentration intracellulaire de calcium, elle subit un changement conformationnel qui lui permet de lier et d'activer diverses cibles protéiques cellulaires. Ce changement structural est similaire à celui qui est observé pour la troponine C et est essentiel dans la contraction musculaire. La structure aux rayons X a été résolue et montre que la protéine présente deux domaines N- et C-terminaux, comprenant chacun deux sites de fixation de l'ion calcium du type hélice-boucle-hélice, le motif structural le plus commun pour la chélation du calcium, présent dans de nombreuses autres protéines liant le calcium comme, par exemple, la troponine C et la parvalbumine. La structure de ce motif est hautement conservée dans toutes les protéines : une boucle de 12 acides aminés est encadrée par deux hélices alpha ; la boucle coordonne le métal via des résidus d'acide aspartique et glutamique, ainsi que par un groupement carbonyle du squelette peptidique et par une molécule d'eau. La structure détaillée du site I de la calmoduline de *Paramecium tetraurelia* (figure 1) montre que le calcium est dans une géométrie en bipyramide à base pentagonale ; les distances Ca-O se situent entre 2,1 et 2,3 Å et les constantes de dissociation sont de l'ordre de 10 $\mu$M pour les sites I et II à basse affinité, et de 1 $\mu$M pour les sites III et IV à haute affinité. Une coopération entre ces sites a été mise en évidence : la liaison du calcium aux sites I et II augmente fortement l'affinité des sites III et IV. La séquence de la boucle chélatante est très conservée dans la calmoduline. En fait, l'homme et la plupart des vertébrés ont la même séquence dans cette boucle.

A partir de la séquence du motif hélice-boucle-hélice des sites de fixation du calcium de la calmoduline de *Paramecium tetraurelia,* les Inventeurs ont préparé des peptides mutants possédant les propriétés suivantes :

- ils présentent - sous forme de peptide isolé et en présence de certains métaux - une conformation ordonnée compatible avec une structure en hélice-boucle-hélice, et

- ils lient - sous forme de peptide isolé ou de protéine incluant au moins une séquence dudit peptide - sélectivement l'uranium dans sa forme uranyle avec une affinité élevée (Kd de l'ordre du $\mu$M), et ce même en présence d'autres métaux, de tampons variés ou d'autres ions physiologiques ou naturels, possédant des caractéristiques de coordination proches de celles de l'uranium.

De tels peptides ou les protéines incluant une ou plusieurs de ces séquences peptidiques ont des applications dans :

- la production de nouvelles bactéries et de nouvelles espèces végétales modifiées exprimant des protéines enrichies en cette séquence peptidique douées d'une capacité accrue de fixation, d'absorption et d'accumulation de ce métal toxique, utiles pour la décontamination biologique des sols et fluides contaminés par l'uranium,
- la fabrication de nouveaux senseurs fluorescents peptidiques et protéiques de l'uranium, utiles pour la détection spécifique de l'uranium, notamment chez des individus susceptibles d'être contaminés,
- la production de nouveaux microorganismes modifiés qui utilisent le peptide spécifique de l'uranium ou la protéine calmoduline, incluant de un à quatre sites sélectifs de l'uranium, en tant qu'élément sensible pour traduire la présence de l'uranium en un signal lumineux, et
- la formulation de nouveaux médicaments pour le traitement des personnes contaminées par l'uranium.

En conséquence, la présente invention a pour objet un peptide mutant du site I de fixation du calcium de la calmoduline défini par l'une des séquences suivantes : SEQ ID NO: 4, 5, 6, 7, 9, 10 et 12.

Le peptide de l'invention est caractérisé en ce qu'il possède une structure du type hélice-boucle-hélice comprenant au moins une mutation au niveau de la boucle du motif hélice-boucle-hélice. Les mutations des peptides de l'invention sont illustrées au Tableau I :

- D24T (peptide CaM-M10c ; SEQ ID NO : 12)
- D20T et D24T (peptide CaM-M3c ; SEQ ID NO : 4)
- D20S et D24S (peptide CaM-M7c; SEQ ID NO: 9)
- D20T et D22T (peptide CaM-M6c ; SEQ ID NO : 7)
- D22T et D24T (peptide CaM-M5c ; SEQ ID NO : 6)
- D20N, D22N et D24N (peptide CaM-M4c ; SEQ ID NO : 5)
- D20T, D22T et D24T (peptide CaM-M8c ; SEQ ID NO : 10).

Conformément à l'invention, pour obtenir un complexe de coordination dans lequel l'uranyle a un nombre de coordination de 7 (géométrie bipyramidale à base pentagonale ou hexagonale), de manière à fournir à l'uranyle, en plus des deux atomes d'oxygène en position apicale, 5 ou 6 atomes coordinants placés aux quatre sommets de la « base carrée » de la bipyramide, le peptide selon l'invention est modifié par rapport au peptide naturel correspondant, pour que le nombre de résidus d'acides carboxyliques présents dans la boucle (D20, D22 et D24, E31) soit diminué de telle sorte qu'il soit inférieur à 4 en substituant au moins le résidu 24 ou deux des autres résidus par un ou deux résidus neutres.

Sauf indication contraire, les positions des mutations sont indiquées en référence à la séquence de la calmoduline humaine (SWISSPROT P02593).

Au sens de la présente invention, ladite boucle est celle du site I de fixation du calcium de la calmoduline, définie par la séquence de 12 acides aminés, située des positions 20 à 31, en référence à la séquence humaine (SWISSPROT P02593).

Ledit peptide dérive totalement de la calmoduline, il comprend les séquences des hélices adjacentes à ladite boucle qui sont présentes dans le site I de liaison du calcium, à savoir les séquences correspondant à celles situées aux positions 7 à 19 et 29 à 38, en référence à la séquence humaine (SWISSPROT P02593).

Ledit peptide dérive totalement du site I de la calmoduline et possède les mutations F19C et V35C ; des peptides cycliques possédant de telles mutations et après fixation de certains métaux présentent avantageusement une structure ordonnée du type hélice-boucle-hélice lorsqu'ils sont sous forme de peptides isolés.

Ledit peptide inclut également la mutation T26Y, une mutation du résidu T26 en un résidu d'acide aminé fluorescent, sensible aux variations de l'environnement chimique induites par la fixation de l'uranyle, ledit résidu étant situé dans les hélices à une distance inférieure à 20 Å du site de fixation de l'uranyle.

Selon un autre mode de réalisation avantageux dudit peptide, il est conjugué avec au moins un fluorophore approprié, tel que le dansyle, la coumarine, la fluorescéine et les dérivés Alexa au niveau d'un résidu d'acide aminé approprié, par exemple au niveau des positions 15 et 16 sensibles aux variations conformationnelles et de l'environnement chimique, induites par la fixation de l'uranyle audit peptide marqué par un fluorophore.

Selon un autre mode de réalisation avantageux dudit peptide, il est couplé à deux fluorophores différents, respectivement un donneur de fluorescence et un accepteur de fuorescence, à des positions induisant des variations conformationnelles favorables à un transfert d'énergie du donneur vers l'accepteur de fluorescence (rapprochement des fluorophores), lors de la fixation de l'uranyle audit peptide marqué par deux fluorophores.

Parmi les fluorophores, on peut utiliser des protéines fluorescentes, notamment le GFP et ses mutants (EBFP, ECFP, EYFP, EGFP), ainsi que d'autres molécules fluorescentes, dérivées d'organismes marins, comme par exemple le DsRed, une protéine fluorescente rouge du corail tropical *Dixosoma* (Bowen B. et al., Photochem. Photobiol., 2003, 77, 4, 362-369), le CopGFP, une protéine fluorescente verte (*green monomeric GFP-like protein*), distribuée notamment par Evrogen (www.evrogen.com) ou le PhiYFP, une protéine fluorescente jaune (*yellow fluorescent protein*) distribuée notamment par Evrogen (www.evrogen.com).

Selon encore un autre mode de réalisation avantageux dudit peptide, il est associé, par tout moyen approprié, à des molécules permettant le ciblage dans le rein et/ou dans les os, par exemple des molécules scFv spécifiques, des facteurs de croissance spécifiques ou des peptides spécifiques.

Selon encore un autre mode de réalisation avantageux dudit peptide, il est associé, par tout moyen approprié, à des molécules favorisant son excrétion *in vivo*, par exemple à des molécules de polyéthylène glycol.

La présente invention a également pour objet un polypeptide caractérisé en ce qu'il comprend la concaténation d'au moins deux peptides identiques ou différents, tels que définis ci-dessus.

De tels polypeptides augmentent l'affinité pour l'uranium, du fait de la coopération entre différents sites de liaison.

La présente invention a également pour objet une composition peptidique, caractérisée en ce qu'elle comprend un polypeptide tel que défini ci-dessus, comprenant la concaténation d'au moins deux peptides identiques ou différents, tels que définis ci-dessus, associés entre eux par tout moyen approprié et au moins un véhicule convenable.

La présente invention a également pour objet une protéine de fusion, caractérisée en ce qu'elle est constituée par la fusion en phase de la séquence d'au moins un peptide tel que défini ci-dessus avec la séquence d'une protéine appropriée.

Conformément à l'invention ledit peptide est fusionné au niveau d'un site permissif de ladite protéine, c'est-à-dire au niveau d'une région de cette protéine qui, lorsqu'elle est fusionnée à la séquence d'au moins un peptide selon l'invention, confère à ladite protéine de fusion une affinité et une spécificité pour l'uranium VI, de l'ordre de celle du peptide isolé.

Parmi les protéines appropriées, on peut citer les protéines possédant un motif hélice-boucle-hélice, capables de fixer le calcium, notamment la calmoduline et les protéines issues des précédentes, notamment les senseurs « caméléons », selon le principe tel que décrit dans Miyaki et al. et Truong et al., précités.

Par exemple, lorsque ladite protéine est la calmoduline ou une protéine caméléon dérivée de la précédente, la séquence dudit peptide est insérée en lieu et place de la séquence correspondante dans la calmoduline, à savoir à la place de la boucle ou du motif hélice-boucle-hélice natifs (non-mutés).

Selon un mode de réalisation avantageux de ladite protéine de fusion elle est conjuguée, par tout moyen approprié à au moins un fluorophore, tel que défini ci-dessus. De préférence, l'une des extrémités de la dite protéine est couplée à un donneur de fluorescence, et l'autre extrémité est couplée à un accepteur de fluorescence. De manière préférée, ladite protéine comprend à l'une de ses extrémités la séquence de l'EBFP ou l'ECFP et à l'autre extrémité la séquence de l'EGFP ou de l'EYFP.

Les peptides, les polypeptides et les protéines fluorescents tels que définis ci-dessus qui présentent une affinité élevée et une spécificité pour l'uranium, représentent des senseurs fluorescents spécifiques de l'uranium VI utiles pour la détection et le dosage de l'uranium dans les sols et les eaux contaminés, ainsi que dans un échantillon biologique approprié, notamment un échantillon de fluide corporel, issu d'un individu susceptible d'être contaminé par l'uranium.

En conséquence, la présente invention a également pour objet l'utilisation d'un peptide, d'un polypeptide ou d'une protéine de fusion, tels que définis ci-dessus, pour la préparation d'un réactif destiné à la détection des sols et des eaux contaminés par l'uranium, ainsi que pour le diagnostic d'individus contaminés par l'uranium. De préférence, lesdits peptides et lesdites protéines de fusion sont conjugués à au moins un fluorophore, tel que défini ci-dessus.

La présente invention a également pour objet l'utilisation d'un peptide, d'un polypeptide ou d'une protéine de fusion, tels que définis ci-dessus, pour la préparation d'un médicament destiné au traitement des individus contaminés par l'uranium.

Conformément auxdites utilisations, lesdites protéines capables de lier le calcium sont notamment la troponine C ou la parvalbumine. Dans de tels cas, le peptide mis en oeuvre, correspond soit à la protéine entière, soit il contient des mutations d'au moins un résidu de chaque hélice en un résidu de cystéine, de façon à obtenir un peptide cyclique présentant une structure ordonnée du type hélice-boucle-hélice.

Les peptides selon l'invention sont préparés par les techniques classiques de synthèse en phase solide ou liquide, connues en elles-mêmes de l'Homme du métier. Les protéines selon l'invention sont préparées par les techniques d'ADN recombinant, connues en elles-mêmes de l'Homme du métier.

En conséquence, la présente invention a également pour objet une molécule d'acide nucléique isolée, caractérisée en ce qu'elle comprend une séquence codant pour un peptide, un polypeptide ou une protéine de fusion, tels que défini ci-dessus.

Les molécules d'acide nucléique selon l'invention sont obtenues par des méthodes classiques, connues en elles-mêmes, en suivant les protocoles standards tels que ceux décrits dans Current Protocols in Molecular Biology (Frederick M. AUSUBEL, 2000, Wiley and son Inc, Library of Congress, USA).

Les séquences codant pour un peptide, un polypeptide ou une protéine selon invention peuvent être obtenues par amplification d'une séquence nucléique par PCR ou RT-PCR ou bien par criblage de banques d'ADN génomique par hybridation avec une sonde homologue. Par exemple, elles sont amplifiées par PCR à l'aide d'une paire d'amorces appropriée telle que définie ci-dessus.

La présente invention a également pour objet un vecteur recombinant eucaryote ou procaryote, caractérisé en ce qu'il comprend un insert constitué par une molécule d'acide nucléique codant pour un peptide, un polypeptide ou une protéine de fusion tels que définis ci-dessus. De nombreux vecteurs dans lesquels on peut insérer une molécule d'acide nucléique d'intérêt afin de l'introduire et de la maintenir dans une cellule hôte eucaryote ou procaryote, sont connus en eux-mêmes ; le choix d'un vecteur approprié dépend de l'utilisation envisagée pour ce vecteur (par exemple réplication de la séquence d'intérêt, expression de cette séquence, maintien de la séquence sous forme extrachromosomique ou bien intégration dans le matériel chromosomique de l'hôte), ainsi que de la nature de la cellule hôte. Par exemple, on peut utiliser des vecteurs viraux ou non-viraux comme des plasmides.

De préférence, ledit vecteur recombinant est un vecteur d'expression dans lequel ladite molécule d'acide nucléique ou l'un de ses fragments sont placés sous le contrôle d'éléments régulateurs de la transcription et de la traduction appropriés. En outre, ledit vecteur peut comprendre des séquences (étiquettes ou *tag*) fusionnées en phase avec l'extrémité 5' et/ou 3' dudit insert, utiles pour l'immobilisation, et/ou la détection et/ou la purification de la protéine de fusion, du peptide ou du polypeptide exprimés à partir dudit vecteur.

Ces vecteurs sont construits et introduits dans des cellules hôtes par les méthodes classiques d'ADN recombinant et de génie génétique, qui sont connues en elles-mêmes.

La présente invention a également pour objet des cellules eucaryotes ou procaryotes modifiées par un vecteur recombinant tel que défini ci-dessus.

La présente invention a également pour objet un organisme animal non-humain transgénique, caractérisé en ce qu'il comprend des cellules modifiées par une molécule d'acide nucléique telle que définie ci-dessus.

La présente invention a également pour objet une plante transgénique, caractérisée en ce qu'elle comprend des cellules modifiées par une molécule d'acide nucléique telle que définie ci-dessus.

Les cellules procaryotes ou eucaryotes modifiées par une molécule d'acide nucléique telle que définie ci-dessus et les plantes transgéniques telles que définies ci-dessus, qui expriment des protéines enrichies en la séquence peptidique selon l'invention, sont douées d'une capacité accrue de fixation, d'absorption et d'accumulation de l'uranium ; elles sont donc utiles pour la décontamination des sols et des fluides contaminés par l'uranium.

En conséquence, la présente invention a également pour objet l'utilisation des cellules procaryotes ou eucaryotes modifiées par une molécule d'acide nucléique telle que définie ci-dessus et des plantes transgéniques telles que définies ci-dessus pour la décontamination des sols et des eaux contaminées par l'uranium.

Les cellules transformées, telles que définis ci-dessus, sont également utiles notamment pour la production du peptide, du polypeptide ou de la protéine de fusion tels que définis ci-dessus.

La présente invention a également pour objet un kit pour la détection d'une contamination par l'uranium, caractérisé en ce qu'il comprend au moins : un peptide, un polypeptide ou une protéine de fusion tels que définis ci-dessus.

Outre les dispositions qui précèdent, l'invention comprend encore d'autres dispositions qui ressortiront de la description qui va suivre, qui se réfèrent à des exemples de mise en oeuvre des antigènes et des anticorps selon la présente invention ainsi qu'au tableau I résumant les séquences de la Demande, dans lequel les résidus de la calmoduline qui ont été mutés sont indiquées en gras et soulignés, et aux dessins annexés dans lesquels :

- la figure 1 illustre en haut la structure tridimensionnelle du site I de la calmoduline de *Paramecium tetraurelia* et la coordination d'un ion calcium ; en bas, des résidus coordonnant le calcium dans les quatre sites,
- la figure 2 illustre les séquences des peptides dérivés du site I de la calmoduline qui ont été étudiés,
- la figure 3 illustre les spectres de dichroïsme circulaire d'une solution 50 $\mu$M du peptide cyclique CaM1c en présence d'ions calcium dans MES 10 mM (pH 6,5), ajoutés à des concentrations égales à 0, 0,2, 0,5, 1,0, 2,0, 5,0, 20 équivalents,
- la figure 4 illustre les variations de l'intensité de dichroïsme circulaire à 222 nm du peptide CaM-M1c, en fonction de la concentration molaire de calcium et son adaptation à la courbe d'isotherme de liaison,
- la figure 5 illustre les variations de l'intensité de fluorescence au maximum d'émission en fonction de la concentration molaire des ions Terbium (à 545 nm) et Europium (à 618 nm), ajoutés au peptide CaM-M1c. Les courbes représentent les isothermes de liaison adaptées aux points expérimentaux,
- la figure 6 représente le diagramme de spéciation de l'uranium (VI) à une concentration de 1 $\mu$M,
- la figure 7 est une représentation schématique de l'uranyle dans la boucle coordinante de la calmoduline mutée (peptide CaM-M3c), obtenue à partir de la structure tridimensionnelle de la boucle de la calmoduline (code PDB :

1EXR),

- la figure 8 illustre les spectres dichroïques d'une solution du peptide CaM-M3c 20 μM dans le tampon MES 1 mM, en présence de 8 équivalents de divers métaux,
- la figure 9 illustre les spectres de fluorescence correspondant au titrage par le peptide CaM-M3c, d'une solution 2,0 μM de nitrate d'uranyle dans l'eau à pH 6,5,
- la figure 10 illustre les spectres de fluorescence correspondant au titrage par le peptide CaM-M3c, d'une solution 2,0 μM de nitrate d'uranyle en tampon phosphate 1 mM, pH 6,5,
- la figure 11 illustre la variation de l'intensité d'émission de fluorescence (à 520 nm) d'une solution 2,0 μM de nitrate d'uranyle en tampon phosphate 1,0 mM, en fonction de la quantité de peptide CaM-M3c et son adaptation avec la relative équation d'isotherme de liaison,
- la figure 12 illustre la diminution de fluorescence de solutions de nitrate d'uranyle 20,0, 2,0 et 0,2 μM en présence d'ions métalliques calcium 2,0 mM, magnésium 2,0 mM et sodium 0,4 mM (et de tampon phosphate 1,0 mM), en fonction de la quantité de peptide CaM-M3c. Cette expérience mime un titrage de l'uranium présent comme contaminant dans une eau de source,
- la figure 13 illustre les spectres de titrage par la protéine calmoduline, d'une solution 2,0 μM de nitrate d'uranyle en tampon phosphate 1,0 mM (pH 6,5),
- la figure 14 illustre le titrage par SLRT d'une solution de nitrate d'uranyle 0,4 μM dans un tampon phosphate 1,0 mM (pH 7,0) par une solution de Calmoduline (0 à 8 μM), puis titrage du mélange final par du calcium (de 0 à 8 mM). Chaque point représente la valeur de l'intensité relevée au maximum du spectre de l'ion uranyle libre en solution corrigée par les variations de l'intensité du tir LASER.
- la figure 15 illustre les spectres de fluorescence du peptide ligand de la calmoduline en l'absence de la protéine (courbe à traits), en présence de 1 équivalent de calmoduline (courbe continue), avec calcium (courbe à traits et points) ou uranium (courbe pointillée).
- la figure 16 illustre la simulation des points expérimentaux obtenus dans l'expérience de titrage d'une solution d'uranyle 0,4 μM par la calmoduline, en utilisant le logiciel Dynafit. Quatre simulations ont été proposées au logiciel en prenant en compte de 1 à 4 sites à haute affinité pour l'uranium par molécule de protéine. Seule la courbe correspondant à 2 sites est représentée ici car elle seule conduit à une simulation correcte de l'expérience et
- la figure 17 correspond à la simulation des points expérimentaux obtenus dans l'expérience de compétition entre calcium et uranyle pour la calmoduline, obtenue par le logiciel Dynafit, en considérant que chaque monomère de protéine calmoduline contient deux sites indépendants l'un de l'autre.

## Tableau I : Liste des peptides étudiés dans la Demande

| N° d'identification | Séquence | Nom |
|---|---|---|
| SEQ ID NO : 1 | EQIAEFKEAFALFDKDGDGTITTKELGTVMRSL | CaM |
| SEQ ID NO : 2 | EQIAEFKEAFALCDKDGDGYITTKELGTCMRSL | CaM-M1c |
| SEQ ID NO : 3 | EQIAEFKEAFALCDKDGDGYITTKDLGTCMRSL | CaM-M2c |
| SEQ ID NO : 4 | EQIAEFKEAFALCTKDGTGYITTKELGTCMRSL | CaM-M3c |
| SEQ ID NO : 5 | EQIAEFKEAFALCNKNGNGYITTKELGTCMRSL | CaM-M4c |
| SEQ ID NO : 6 | EQIAEFKEAFALCDKTGTGYITTKELGTCMRSL | CaM-M5c |
| SEQ ID NO : 7 | EQIAEFKEAFALCTKTGDGYITTKELGTCMRSL | CaM-M6c |
| SEQ ID NO : 8 | RRKWQKTGHAVRAIGRL | MLCKp |
| SEQ ID NO :9 | EQIAEFKEAFALCSKDGSGYITTKELGTCMRSL | CaM-M7c |
| SEQ ID NO :10 | EQIAEFKEAFALCTKTGTGYITTKELGTCMRSL | CaM-M8c |
| SEQ ID NO:11 | EQIAEFKEAFALCTKDGDGYITTKELGTCMRSL | CaM-M9c |
| SEQ ID NO: 12 | EQIAEFKEAFALCDKDGTGYITTKELGTCMRSL | CaM-M10c |

## Exemple I : Matériels et Méthodes

### 1) Synthèse peptidique

Les peptides ont été synthétisés en phase solide, avec un synthétiseur automatique de peptides Applied Biosystems, mod. 433A, et en chimie Fmoc, qui utilise le groupement fluorénylméthyloxycarbonyle (Fmoc) pour la protection temporaire de la fonction α-aminique des acides aminés. Les groupements protecteurs utilisés pour les chaînes latérales des acides aminés ont été le tertio-butyle ether (tBu) pour les résidus Ser, Thr et Tyr ; tertio-butyle ester (OtBu) pour Asp, Glu ; trityle (Trt) pour Gln, Asn, Cys ; tertio-butyloxycarbonyle (Boc) pour Lys et 2,2,5,7,8-penta-métylchromane-6-sulfonyle (Pmc) pour Arg.

La réaction de couplage a été effectuée en présence d'un excès de 10 équivalents d'acide aminé (1 millimole) par rapport à la résine (0,1 millimole). Celle-ci a d'abord été déprotégée du groupement Fmoc par une solution de pipéridine 20 %. La pipéridine en excès a été éliminée par lavage au N-méthylpyrrolidone (NMP). Le suivi de la réaction de déprotection a été effectuée par détection UV à 305 nm des adduits dibenzofulvène-pipéridine. Parallèlement, l'acide aminé a été dissous dans un mélange constitué de 1 ml de NMP et de 1 ml d'une solution 1 M de 1-N-hydroxy-7-azabenzotriazole (HOAt) dans du NMP. Une solution de 1 ml de N,N'-dicyclohexyl-carbodiimide (DCC) 1 M dans NMP a alors été ajoutée pour former l'ester activé de l'acide aminé. Après 40 minutes, cet ester actif a été introduit dans le réacteur contenant la résine déprotégée.

En fin de synthèse, la résine a été lavée plusieurs fois au dichlorométhane (DCM). Le clivage du peptide et la déprotection des groupements protecteurs des chaînes latérales des acides aminés ont été effectués en conditions acides. La résine a été mise en suspension (100 ml par gramme de résine) dans une solution de 81,5 % d'acide trifluoroacétique (TFA), 5% de phénol, 5% de thioanisole, 5% d'eau, 2,5% d'éthanedithiol et 1% de triisopropylsilane pendant trois heures sous agitation à température ambiante. Après filtration sur fritté, le milieu réactionnel a été précipité à l'éther diisopropylique puis centrifugé. Le culot a été séparé du surnageant et dissous dans du TFA. Après reprécipitation à l'éther et centrifugation, le culot a été à nouveau dissous dans de l'acide acétique 20% puis lyophilisé.

Le produit réactionnel brut obtenu a été purifié une première fois sur colonne préparative Vydac C18 phase inverse (1,0 x 25,0 cm) en utilisant un gradient 0-60% d'acétonitrile en 90 minutes. Le peptide linéaire pur a alors été lyophilisé, puis redissous dans 200 ml d'une solution Tris 100 mM, pH 8,0. Un équivalent d'acide 5,5' dithiobis(2-nitrobenzoïque) a été ajouté de façon à conduire à la formation spécifique du pont disulfure intramoléculaire entre les deux cystéines. Le milieu réactionnel a alors été acidifié puis purifié en utilisant le même protocole que pour le produit brut réactionnel. Les fractions de produit pur ont été regroupées et lyophilisées. La pureté du produit a été confirmée par spectrométrie de masse électrospray.

Des solutions stocks ont été préparées par dissolution dans l'eau, et les concentrations ont été déterminées par spectrophotométrie en utilisant des coefficients d'extinction molaire de 1280 $M^{-1}.cm^{-1}$ pour la tyrosine, 120 $M^{-1}.cm^{-1}$ pour le pont disulfure et de 5690 $M^{-1}.cm^{-1}$ pour le tryptophane.

2) <u>Métaux</u>

Tous les sels métalliques utilisés sont des nitrates (>99,9% de pureté, Aldrich, France). Les solutions mères sont acidifiées à pH=2 avec de l'acide nitrique pour éviter la formation d'hydroxydes.

3) <u>Fluorescence</u>

Les spectres de fluorescence sont enregistrés sur un spectromètre Cary Eclipse (Varian, France) équipé d'un porte-cuve thermostaté. La longueur d'onde d'excitation utilisée est de 280 nm avec des largeurs de fentes de 10 nm en excitation et de 2,5 nm en émission. Les spectres sont enregistrés entre 300 nm et 450 nm dans une cuve de 1 cm de chemin optique.

4) <u>Spectrofluorimétrie Laser à Résolution Temporelle</u> (SLRT)

Un laser Nd-YAG (modèle minilite, CONTINUUM) opérant à 266 nm et délivrant une énergie de 1 mJ en pulse de 4 ns à une fréquence de 20 Hz a été utilisé comme source d'excitation. Le faisceau a été dirigé dans une cuve en quartz de 4 ml, puis dans la cellule de mesure du spectrofluorimètre «FLUO 2001 » (Dilor, France) à l'aide de lentilles en quartz. La lumière a alors été concentrée à l'entrée d'un polychromateur, et la détection du signal a été effectuée à l'aide d'une barette de 1024 photodiodes refroidies par effet Peltier (-30°C). L'enregistrement des spectres a été réalisé en intégrant le signal détecté par les photodiodes pendant une durée de 0,5s. Un circuit électronique synchronisé avec le LASER a permis d'effectuer la détection après un délai de 90 μs pendant une durée de 50μs. L'ensemble a été contrôlé par un ordinateur (DELL).

5) <u>Spectrométrie de masse du type électrospray</u> (ESI-MS)

Les spectres de masse du type electrospray en mode de détection positive ont été enregistrés avec un appareil Q-TOF II (MICROMASS). L'échantillon à analyser a été introduit dans la source via un pousse-seringue (HARVARD APPARATUS). L'azote a été utilisé comme gaz de séchage et de collision avec une source chauffée à 80°C. La tension de cône était de 30 Volts , et une haute tension de 3500 kV a été appliquée sur le capillaire. Le débit de solution échantillon a été fixé à 5 μL.min$^{-1}$. Les spectres représentent la moyenne de 40 scans enregistrés entre 400 et 3000 m/z à une vitesse de scan de 6 s/scan.

6) Dichroïsme circulaire (CD)

Les spectres CD ont été enregistrés avec un appareil CD6 (JOBIN YVON) équipé d'un porte cuve thermostaté et contrôlé par un ordinateur utilisant le logiciel CDMax. Les composés ont été solubilisés à une concentration de 5 μM dans un tampon MES 1mM à pH 6,5. Les spectres ont été enregistrés à température ambiante entre 180 nm et 250 nm en utilisant une cuve de 0,1 mm de chemin optique. Chaque spectre représente la moyenne de 4 accumulations successives obtenues avec un temps d'intégration de 0,5 s et un pas de 0,5 nm. Les spectres ont été lissés en utilisant l'algorithme inclus dans le logiciel CDMax.

Exemple 2 : Préparation de peptides cycliques dérivés du site I de la calmoduline, et analyse de la chélation des métaux lourds

1) Préparation de peptides cycliques

Le peptide linéaire de 33 résidus correspondant au site I de la calmoduline (CaM: EQIAEFKEAFALFDKDGDGTITTKELGTVMRSL, SEQ ID NO : 1) testé par dichroïsme circulaire, ne présente aucune structure ordonnée, même mis en présence d'un excès d'ions calcium. A forte concentration (100 μM) il s'agrège en solution, vraisemblablement en raison des interactions intermoléculaires entre les parties hydrophobes des hélices non structurées.

En conséquence, afin d'empêcher ces interactions défavorables à la formation d'une structure hélice-boucle-hélice native et stable, des peptides comprenant un pont disulfure reliant les positions 13 et 29 dudit peptide, correspondant respectivement aux positions 19 et 35 dans la séquence de la calmoduline ont été préparées. En conséquence, des peptides comprenant les mutations Phe19Cys et Va135Cys ont été synthétisés. De plus, la mutation Thr26Tyr a été insérée pour permettre d'introduire une sonde fluorescente dans la boucle coordinante, de façon à suivre la fixation du métal. En outre, l'acide glutamique en position 25 du peptide ou en position 31 de la calmoduline a éventuellement été muté en acide aspartique (Glu31Asp).

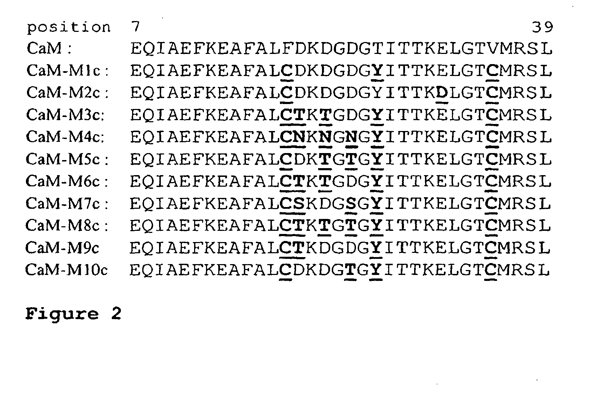

Les peptides synthétisés présentent les séquences suivantes (figure 2 et Tableau I), dans lesquelles les mutations sont indiquées en gras :

CaM-M1c : EQIAEFKEAFAL**C**DKDGDG**Y**ITTKELGT**C**MRSL (SEQ ID NO : 2)
CaM-M2c : EQIAEFKEAFAL**C**DKDGDG**Y**ITTK**D**LGT**C**MRSL (SEQ ID NO : 3)

**2) Analyse de la structure et de l'affinité des peptides cycliques pour les métaux lourds (peptides CaM-M1e et CaM-M2c)**

a) Peptide CaM-M1c

Le peptide CaM-M1c correspondant a été synthétisé et son affinité vis-à-vis de divers métaux testée par spectrométrie de masse, dichroïsme circulaire (DC) et fluorescence à résolution temporelle (SLRT). Les spectres DC enregistrés en présence de 8 équivalents de métaux montrent une bonne affinité pour le calcium, le cadmium, le terbium, l'europium et l'uranium, ainsi qu'une affinité plus faible avec le cobalt. Aucune interaction n'est détectée avec les autres éléments de la colonne des alcalino-terreux (Mg, Sr, Ba).

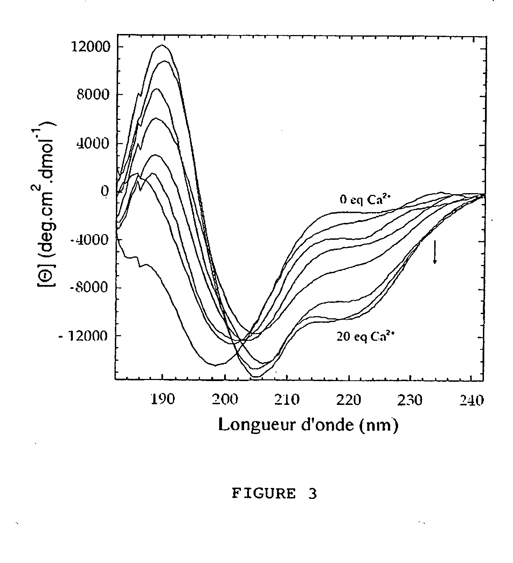

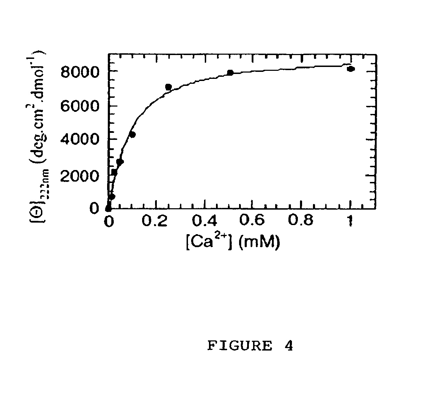

L'analyse par dichroïsme circulaire (DC) du peptide CaM1c montre un spectre typique d'une structure désordonnée avec un minimum à 190 nm (figure 3). L'absence de structure secondaire a été confirmée par spectroscopie de résonance magnétique nucléaire du proton (1H RMN). Par l'addition de calcium en solution, le spectre DC prend une forme qui est typique d'une conformation hélicoïdale avec de minima à 206 et 222 nm. Un titrage de CaM1c par DC a été alors effectué et l'intensité du signal dichroïque à 222 nm a été reportée en fonction de la concentration d'ions calcium ajoutés (figure 4). La courbe d'isotherme de liaison qui passe par les points expérimentaux démontre une stoechiométrie de 1/1 Ca/peptide et permet de calculer une constante de dissociation Kd de 30 μM.

Le spectre de masse du peptide en absence du métal montre trois pics principaux à 736,8, 920,7 et 1227,3 m/z, correspondant au peptide cinq, quatre et trois fois protoné, respectivement. L'introduction des concentrations croissantes en calcium au peptide mène à une modification de ce spectre avec de nouveaux pics à 744,7, 930,6, 1240,5 m/z, compatibles avec un complexe de 1:1 peptide:calcium et présentant le même état de charge. Supposant que le peptide libre en métal et le complexe ont les réponses semblables de signal, une constante de dissociation peut être calculée, comme décrit par Whittal et al. (Prot. Sci., 2000, 9, 332-343), conduisant à une Kd = 30 μM (Tableau II), en accord avec la valeur calculée par le titrage DC.

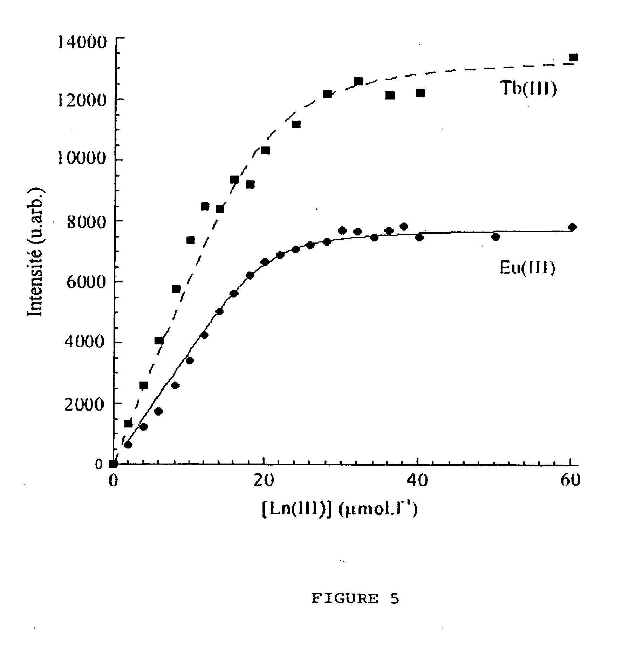

Des ions lanthanides ont été souvent employés comme modèles du calcium dans les études biologiques de molécules (Linse et al., J. Biol. Chem., 1991, 266, 8050-8054). Des titrages de spectrofluorimétrie du peptide CaM-M1c (solution 20 μM dans 10 mM tampon MES, pH 6,5) par des solutions de terbium et d'europium ont été exécutés par fluorescence laser résolue dans le temps (SLRT) (figure 5). Cette spectroscopie est basée sur une excitation du métal, suivie de la résolution temporelle du signal fluorescent, surmontant ainsi les limitations dues à la présence des fluorophores dont la fluorescence est de courte durée de vie mais de forte intensité (Whittal et al., précité). En utilisant une longueur d'onde d'excitation de 266 nm, l'émission de fluorescence des deux lanthanides est observée par un mécanisme de transfert d'énergie par l'intermédiaire de la Tyr20 du peptide, avec une augmentation de la fluorescence émise du métal jusqu'à une limite correspondant à un rapport lanthanide : peptide de 1 : 1. Pour le terbium, l'émission plus forte est située à 545 nm. Dans le cas de l'europium, le spectre montre des maxima d'émission de fluorescence à 593 et 618 nm. Les mesures d'intensité d'émission de fluorescence (545 nm pour Tb3+ et 618 nm pour Eu3+) en fonction de la concentration en lanthanide et l'adaptation de ces données avec l'isotherme de liaison conduisent à déterminer les constantes de dissociation Kd(Tb3+) = 3,5 $\mu$M et Kd(Eu3+) = 0,6. (Tableau II).

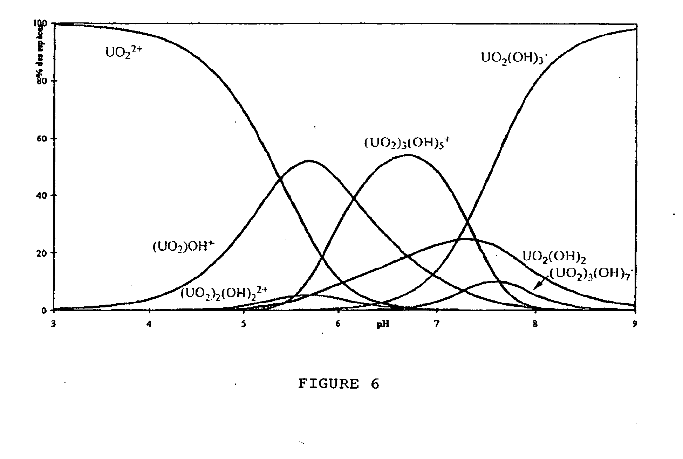

Dans le cas des ions uranyles, l'étude de la fixation d'ions uranyles au peptide est rendue difficile par la spéciation complexe de ce métal dans l'eau (figure 6). En effet, à pH 6,5, le diagramme de spéciation montre que les espèces majoritaires sont $(UO_2)_3(OH)_5^+$ (67%) et $(UO_2)(OH)^+$ (17%). A ce pH, il ne reste que 5% d'ion uranyle $UO_2^{2+}$. L'analyse du complexe formé avec le peptide par spectrométrie de masse montre que l'uranium est coordonné sous la forme $UO_2^{2+}$. Or, toutes les espèces de l'uranium sont fluorescentes, et il devient difficile, par des méthodes de fluorescence classiques, de suivre une seule des espèces présentes en solution. Ce problème a été résolu par la SLRT qui repose sur le principe suivant : après une excitation par une impulsion laser, la détection de la fluorescence est effectuée après un délai choisi par l'utilisateur ; un délai de 80 $\mu$s a permis de s'affranchir de toutes les espèces autre que $UO_2OH^+$ pour la détection. Dans ces conditions instrumentales, en utilisant une longueur d'onde d'excitation de 266 nm, un titrage d'une solution 2 $\mu$M de $UO_2(NO_3)_2$ a été effectué par des ajouts successifs d'aliquotes d'une solution aqueuse du peptide. Cette expérience a permis de déterminer une constante de dissociation $K_d$ de 4,7 $\mu$M, Tableau II.

**Tableau II: Constantes de dissociation des complexes formés avec CaM-M1c en milieu aqueux à pH 6.5**

| Métal | Ca(II) | Cd(II) | U*(VI) | Tb(III) | Eu(III) |
|---|---|---|---|---|---|
| Kd ($\mu$M) | 30 $\pm$ 1[a,c] | 8 $\pm$ 4[a] | 4,7 $\pm$ 0,6[b] | 1,5 $\pm$ 0,6[b] | 0,6 $\pm$ 0,2[b] |
| a. Titrage par DC, b. Titrage par SLRT, c. Titrage par ESI/MS<br>* L'espèce titrée est l'entité $UO_2(OH)^+$. | | | | | |

b) Peptide CaM-M2c

Un deuxième peptide comprenant une mutation additionnelle, à savoir: la substitution de l'acide glutamique en position 31 de la séquence de la calmoduline en acide aspartique (Glu31Asp), a également été synthétisé. La chaîne latérale de l'acide aminé est raccourcie d'un groupement méthylénique, et la cavité formée par la boucle a donc une taille plus importante. Les mêmes études de ESI/MS, DC, SLRT montre que ce peptide perd l'affinité pour tous les métaux divalents, ainsi que pour l'ion uranyle. Seules sont conservées les affinités pour les lanthanides avec des constantes de dissociation de 3,5 $\pm$ 1 $\mu$M et 3,2 $\pm$ 0,8 $\mu$M pour le Terbium et l'Europium respectivement.

L'ensemble des résultats montre que les peptides cycliques étudiés contenant les mutations Phe19Cys, Va135Cys et Thr26Tyr et éventuellement la mutation Glu31Asp, et dans lesquels les cystéines 19 et 35 sont reliées par un pont didulfure, présentent les propriétés suivantes :

- contrairement au peptide linéaire correspondant au site I de la calmoduline (peptide CaM) qui ne présente pas de structure ordonnée et s'agrège en solution, les peptides cycliques synthétisés possèdent une structure stable du type hélice-boucle-hélice, et
- ils sont capables de lier de ions métalliques dont l'uranium VI (peptide CaM-M1c), avec une affinité comparable à celle de la calmoduline native pour l'ion calcium.

Ces résultats indiquent également que des mutations ponctuelles de la séquence de la boucle du site I de la calmoduline permettent de faire varier l'affinité relative des peptides pour divers ions métalliques. Toutefois, aucun des peptides mutants étudiés ne lie spécifiquement l'uranium VI.

**Exemple 3 : Préparation de peptides cycliques spécifiques de l'uranium VI.**

**1) Synthèse des peptides**

Les peptides synthétisés correspondent à des peptides cycliques contenant les mutations Phe19Cys, Va135Cys et Thr26Tyr, telles que décrites à l'exemple 2 ainsi que les mutations additionnelles suivantes :

- D20T (peptide CaM-M9c)
- D24T (peptide CaM-M10c)
- D20T et D24T (peptide CaM-M3c)
- D20S et D24S (peptide CaM-M7c)
- D20T et D22T (peptide CaM-M6c)
- D22T et D24T (peptide CaM-M5c)
- D20N, D22N et D24N (peptide CaM-M4c)
- D20T, D22T et D24T (peptide CaM-M8c)

De manière plus précise, les séquences de ces peptides, dans lesquelles les résidus mutés sont indiqués en gras, sont représentées à la figure 2 et au Tableau I.

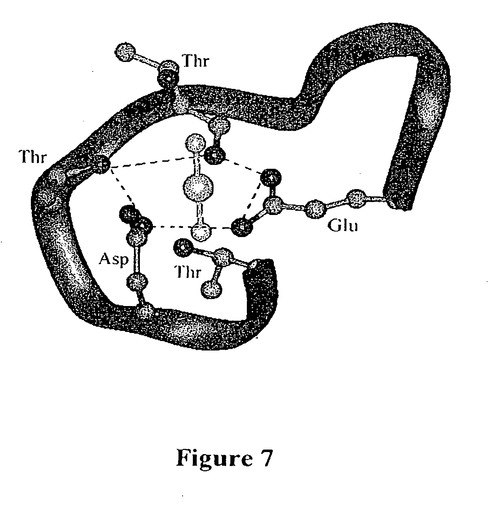

Une représentation schématique de l'uranyle dans la boucle coordinante de la calmoduline mutée (peptide CaM-M3c), obtenue à partir de la structure tridimensionnelle de la boucle de la calmoduline (code PDB : 1EXR) est présentée dans la figure 7.

**2) Analyse de l'affinité des peptides pour différents ions métalliques (peptides CaM-M3c, CaM-M4c et CaM-M5c).**

L'affinité du peptide CaM-M3c pour différents ions métalliques ($Ca^{2+}$, $Mg^{2+}$, $Ba^{2+}$, $Sr^{2+}$, $Tb^{3+}$, $Eu^{3+}$, $UO_2^{2+}$) a été testée en utilisant deux méthodes spectroscopiques : le dichroïsme circulaire (DC) et la spectrométrie de masse électrospray (ESI-MS).

Les spectres DC et les spectres de masse electrospray (ESI-MS) en mode de détection positive ont été enregistrés comme décrit à l'exemple 1.

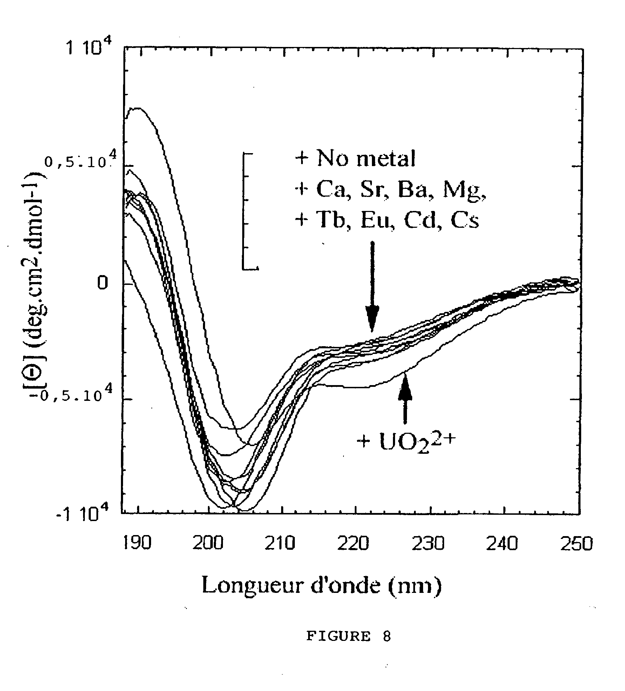

La figure 8 montre que seul l'ajout d'un excès d'uranyle conduit à une modification du spectre dichroïque du peptide. CaM-M3c. Dans ce cas, deux nouveaux minimas à 207 nm et 222 nm sont observés. Ils sont caractéristiques d'une structure secondaire ordonnée en hélice $\alpha$. Ce résultat est confirmé par l'analyse ESI-MS : seul l'ajout d'uranyle en solution conduit à l'apparition d'un pic de masse compatible avec la formation d'un complexe 1/1 peptide/$UO_2$.

Les peptides CaM-M4c, CaM-M5c, CaM-M6c, CaM-M7c, CaM-M8c, CaM-M9c et CaM-M10c ont donné les mêmes résultats en spectroscopie de dichroïsme circulaire et de masse (ESI-MS), ce qui indique que ces peptides ne lient pas le calcium, les lanthanides et les autres ions testés mais seulement l'uranium (VI).

**3) Analyse de la coordination de l'uranium par le peptide CaM-M3c**

a) Problème à résoudre (spéciation)

L'analyse de la coordination de l'ion uranyle par une molécule biologique est envisagée à un pH proche de la valeur physiologique, c'est-à-dire comprise entre 6 et 8. Or, à ce pH, l'ion uranyle ne se présente plus en solution uniquement sous une seule forme $UO_2^{2+}$, mais sous la forme de différents complexes dérivés de ce coeur métallique : complexes hydroxos et carbonates par exemple. Ce phénomène est désigné sous le terme de spéciation. La quantité de chacune des espèces présentes en solution aqueuse dépend de la concentration de l'uranium, de la concentration des gaz dissous (carbonates), ainsi que des paramètres thermodynamiques associés à l'ion métallique. A une concentration de 1 $\mu M$ en uranium, le diagramme de spéciation (figure 6) montre que les espèces majoritaires à pH 6,5 sont les espèces $(UO_2)_3(OH)_5^+$, $UO_2(OH)_2$ et $UO_2(OH)^+$ représentant respectivement 52,1 ; 16 et 25,3 % de l'U(VI) en solution. Les espèces minoritaires sont $UO_2^{2+}$, $UO_2(OH)_3^-$ ainsi que des complexes uranium/carbonates.

Lors d'une expérience de titrage par fluorescence classique, chacune de ces espèces de l'uranium participe à l'intensité globale de fluorescence détectée après excitation. Le calcul d'une constante de dissociation est dès lors impossible puisqu'on ne peut pas isoler la contribution de chacune des espèces présentes en solution. Pour cette raison, les titrages ont été effectués en utilisant la résolution temporelle, c'est-à-dire la différence du temps de vie de fluorescence de chacune des espèces mises en jeu, comme décrit à l'exemple 1. En utilisant un délai supérieur ou égal à 70 $\mu s$ entre le tir laser (excitation) et la détection, la seule espèce détectée est le complexe monohydroxo $UO_2(OH)^+$.

b) Analyse de la coordination de l'uranium par le peptide CaM-M3c dans différents milieux

$b_1$) Analyse de la coordination de l'uranium dans l'eau

L'uranium à 2 $\mu M$ a été titré par le peptide CaM-M3c dans un milieu aqueux pur dont le pH est ajusté à 6,5 à l'aide d'ammoniaque. Les paramètres de la résolution temporelle (70 $\mu s$ de délai, 100 $\mu s$ de largeur de porte, 0,5 s d'intégration) permettent de ne visualiser que l'espèce monohydroxylée de l'uranium.

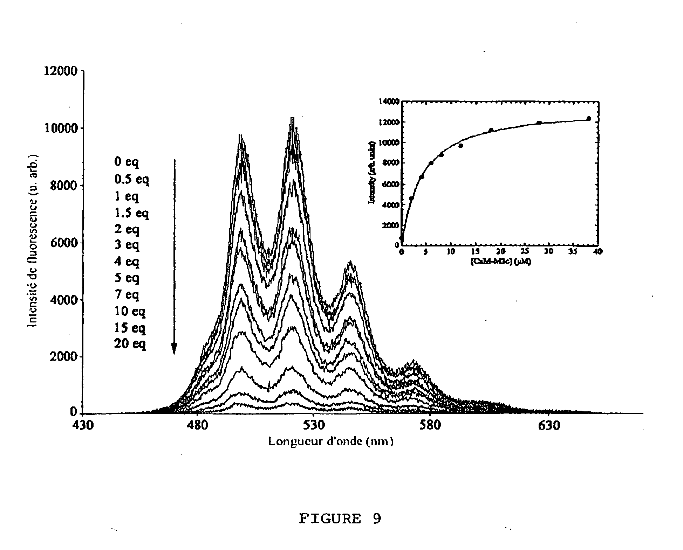

A l'ajout de quantités croissantes de peptide dans la solution initiale d'uranium, l'intensité de la fluorescence

de UO$_2$(OH)+ diminue attestant de la coordination du métal au peptide. Le graphe de l'intensité à 520 nm en fonction de la concentration en peptide ajouté peut être simulé par l'expression théorique correspondant à l'équilibre chimique mis enjeu, en tenant compte du fait que la concentration de UO$_2$(OH)+ en solution est égale à 17,21% de l'U(VI) introduit. La constante de dissociation correspondant à l'équilibre UO$_2$(OH) + CaM-M3c → (CaM-M3c)(UO$_2$) + OH⁻ est calculée à partir de la simulation : Kd = 3,8 $\pm$ 0,3 μM (figure 9).

b$_2$) Analyse, de coordination de l'uranium en milieu phosphate

Dans une deuxième expérience, la chélation de l'uranium par le peptide a été étudiée dans un milieu tampon phosphate 1 mM, à pH 6,5. Dans ce milieu, l'uranyle forme initialement des complexes avec les ions phosphate dont les données thermodynamiques sont les suivantes :

$$PO_4^{3-} + UO_2^{2+} = UO_2PO_4^- \qquad \log_{10}K = 13.7$$

$$HPO_4^{2-} + UO_2^{2+} = UO_2HPO_4 \qquad \log_{10}K = 7.7$$

$$H_3PO_4 + UO_2^{2+} = UO_2H_2PO^+ + H^+ \qquad \log_{10}K = 1.1$$

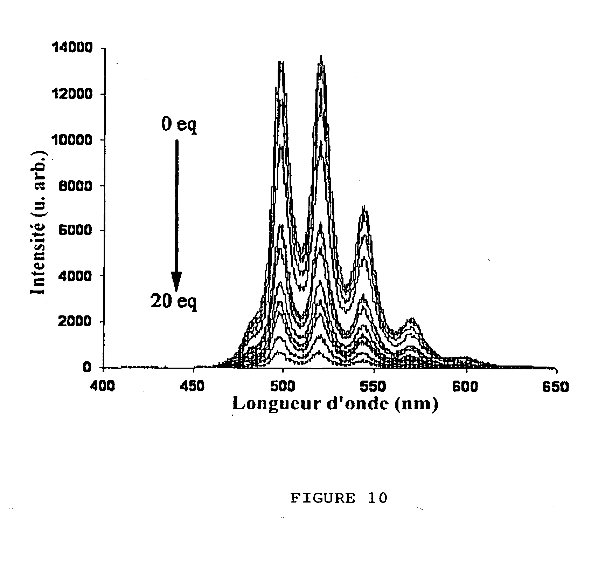

Les complexes uranium/phosphate présentent la particularité d'augmenter l'intensité de fluorescence de l'ion métallique alors que les autres ligands connus conduisent à une atténuation de la fluorescence.
Des fractions d'une solution aqueuse du peptide ont été ajoutés successivement à une solution 2,0 μM de UO$_2$(NO$_3$)$_2$ dans un tampon phosphate 1 mM à pH 6,5. Le spectre de fluorescence de l'ion uranyle ($\lambda_{ex}$ = 266 nm) a été enregistré après chaque ajout. L'ensemble des spectres obtenus est représenté à la figure 10.
Le graphe de l'intensité à 520 nm en fonction de la concentration d'uranium ajoutée est alors simulé par l'isotherme de liaison correspondant à la formation d'un complexe 1/1 entre l'uranium et le peptide, selon la relation, déduite de l'expression de la constante de dissociation :

$$I_0 - I = \frac{(I_0 - I)_{\max}}{4[U]_0}\left[4[U]_0 + \frac{K_d}{[P]} - \sqrt{(4[U]_0 + \frac{K_d}{[P]})^2 - 16[U]_0^2}\right] \qquad (1)$$

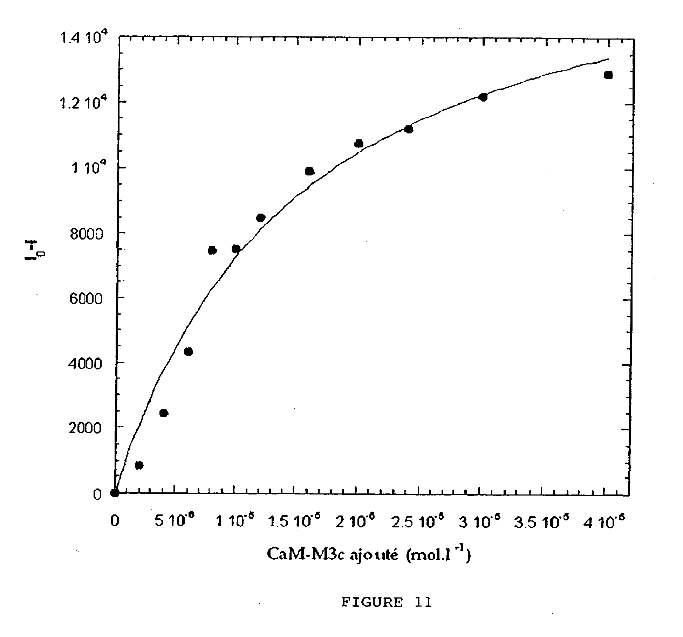

où [U]$_0$ = 2,0 μM, K$_d$ est la constante de dissociation du complexe, et [P] désigne la quantité de peptide ajouté en solution. Les données expérimentales ainsi que son interprétation par l'équation (I) sont représentées à la figure 11. La constante de dissociation calculée par cette approche est de 18 μM.

b$_3$) Analyse de la coordination de l'uranium en présence d'autres ions

La coordination de l'uranium par le peptide a également été étudiée en présence d'un mélange d'autres ions. La composition du milieu réactionnel correspond à une moyenne de la composition en ions de plusieurs eaux de source françaises, auxquelles ont été retirés les ions carbonates qui sont des inhibiteurs de fluorescence de l'uranyle. La composition exacte du milieu testé est la suivante :

[Ca$^{2+}$]=[Mg$^{2+}$]= 2,0 mM
[Na$^+$] = 0,4 mM
[NO$_3^-$] = [SO$_4^{2-}$] = 4,0 mM
pH = 6,5

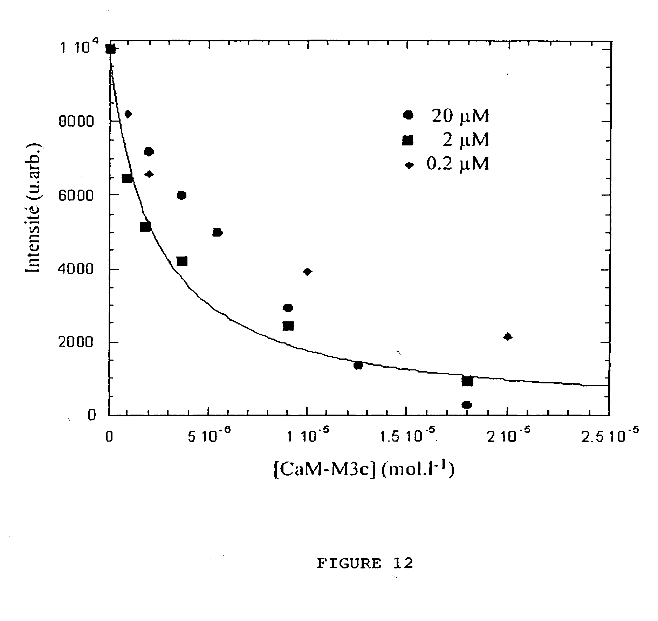

Ce milieu est artificiellement contaminé par des concentrations d'uranium de plus en plus faibles (20 μM, 2 μM, 0,2 μM). Dans chacun des cas, le peptide est ajouté jusqu'à obtenir une extinction totale de la fluorescence de l'uranium (figure 12).
Les constantes de dissociation apparentes calculées pour chacun de ces titrages sont de 10 $\pm$ 1 μM. Elles sont du même ordre de grandeur que la constante de dissociation calculée dans l'eau déionisée à pH 6,5 et en tampon phosphate. Ceci met en évidence l'absence de compétition entre U(VI) et les autres cations métalliques d'une part, et entre le peptide et les autres ligands de l'uranium d'autre part : le peptide CaM-M3c est donc sélectif de l'uranium dans ces conditions.
Les résultats obtenus montrent que le peptide CaM-M3c qui possède les mutations D20T et D24T est sélectif de l'uranium VI. Dans le domaine de concentration étudiée (0,1 μM à 2,0 mM en métaux), le peptide CaM-M3c coordine l'uranium avec une constante de dissociation entre 3,8 et 18 μM dans les différents milieux testés. Aucune affinité mesurable pour Mg, Ca, Sr, Ba, Eu, Tb n'est détectée.

**Exemple 4 : Analyse de l'interaction de l'uranium avec la calmoduline native de cerveau bovin**

**1) Analyse de la coordination de l'uranium par la protéine calmoduline de cerveau de boeuf.**

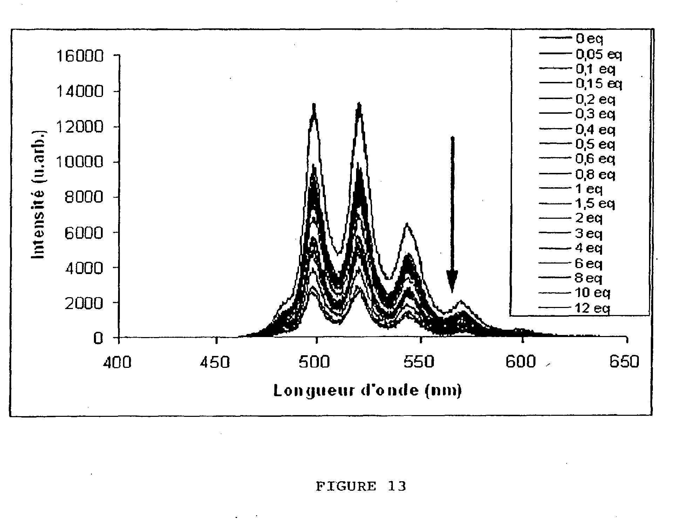

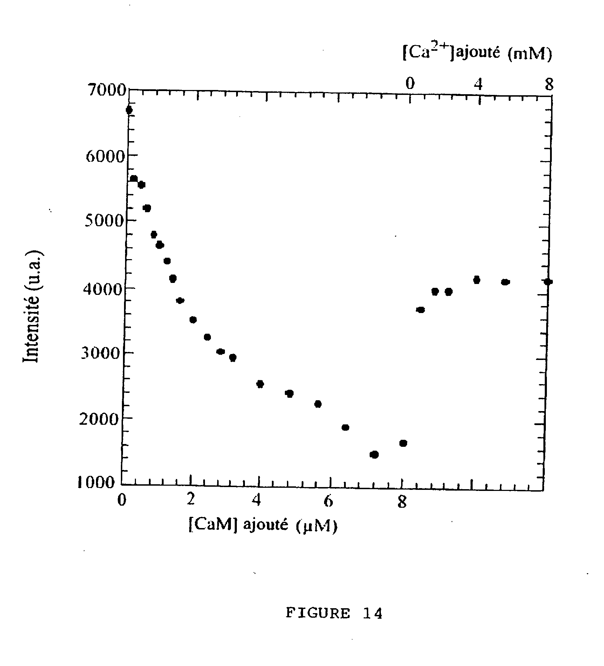

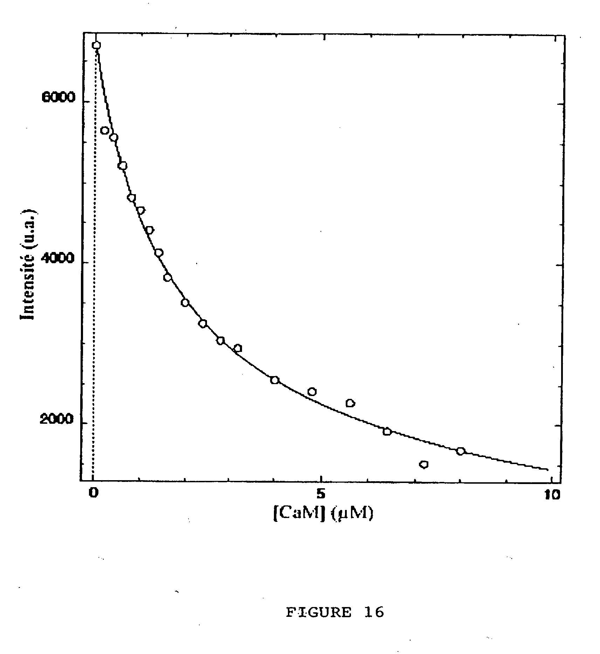

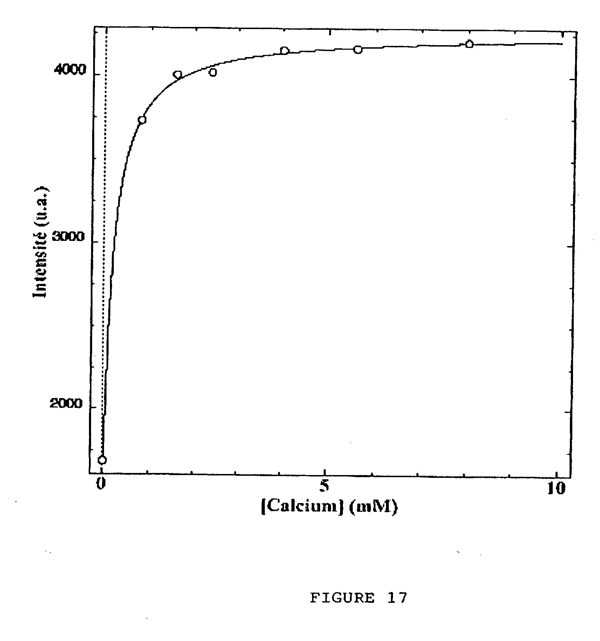

La coordination de l'uranium (sous forme d'uranyle, 2,0 μM) par la protéine native en milieu phosphate 1 mM a également été étudiée. La complexation de l'uranium à la protéine se traduit par une chute de l'intensité de fluorescence de l'uranyle. Les spectres du titrage sont représentés à la figure 13. Dans une autre expérience, une solution de nitrate d'uranyle 0,4 μM dans un tampon phosphate 1,0 mM (pH 7,0) a été titrée par une solution de calmoduline jusqu'à une concentration protéique de 8 μM. Après ajout de calmoduline 8 μM, des ions calcium ont été ajoutés jusqu'à la concentration de 8 mM (figure 14). Les valeurs du titrage de l'uranyle par la calmoduline ont été interprétées en simulant les données expérimentales par un système d'équations correspondant à un, deux, trois ou quatre sites indépendants pour l'uranium (figure 16). Le logiciel Dynafit (Kuzmic, P. 1996, Anal. Biochem. 237, 260-273) a été utilisé pour ces simulations. Seul le système prenant en compte deux sites à haute affinité conduit à une simulation acceptable avec des constantes de dissociation de 3,3 ± 0,4 μM et 0,72 ± 0,2 μM pour chacun des deux sites. Les valeurs de compétition avec les ions calcium ont été également interprétées avec un système d'équations adaptées et la simulation des points expérimentaux avec ce système est reportée dans la figure 17. La simulation a été effectuée en considérant qu'en présence de 8 μM de calmoduline et de 0,4 μM d'uranyle, chacun des deux sites à haute affinité est peuplé de manière équiprobable en uranium. Il est également considéré par hypothèse que chacun de ces deux sites est indépendant de l'autre. La simulation est donc effectuée par un système de quatre équilibres chimiques: deux correspondent aux équilibres de dissociation des complexes « site-uranium », et deux correspondent au déplacement de l'uranium par le calcium dans chacun des sites. La simulation confirme qu'un seul des deux sites complexé à l'uranium est déplacé par le calcium. Ceci montre que la protéine calmoduline peut fixer l'uranium dans des sites de complexation du calcium.

**2) Interaction de la protéine avec son ligand en présence d'uranium.**

La calmoduline, en présence de calcium, peut lier et activer une grande diversité de cibles. Parmi celles-ci, le peptide MLCKp, de 17 résidus et dérivé du domaine liant la calmoduline d'une kinase la chaîne légère de la myosine de muscle de lapin (CALBIOCHEM) présente la séquence suivante :

Ac-RRKWQKTGHAVRAIGRL-NH$_2$ (SEQ ID NO : 8)

Deux séries d'expériences ont été réalisées pour vérifier que la protéine peut toujours interagir avec ce ligand en présence de l'ion uranyle.

Dans une première série, un spectre de fluorescence du tryptophane du ligand, dissous à la concentration 5 μM dans un tampon MES 10 mM à pH 6,5 a été enregistré, comme décrit à l'exemple 1.

L'excitation de la solution à 280 nm permet de détecter une émission à 350 nm, caractéristique d'un tryptophane exposé au solvant aqueux. L'ajout d'un équivalent de calmoduline, en l'absence de métaux, conduit à un déplacement du maximum d'émission à 330 nm ainsi qu'à une augmentation de 25% de l'intensité. Ceci montre que la protéine interagit avec le ligand, le tryptophane de celui-ci se trouvant alors dans un milieu plus hydrophobe. L'ajout en solution de 10 équivalents de calcium conduit à un spectre d'émission de fluorescence qui présente une intensité augmentée (+ 20%) et le même maximum à 330 nm. Ces données sont en accord avec l'obtention d'une structure similaire à la structure cristallographique du complexe peptide/calmoduline obtenue en présence de calcium (PDB 1 CDL).

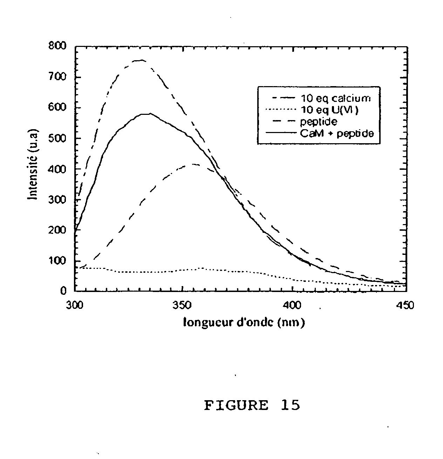

La même expérience réalisé en présence d'uranyle conduit à une extinction totale de la fluorescence du tryptophane du ligand (figure 15). Ceci est compatible avec une structure similaire à celle obtenue dans le cas du calcium. En effet, ce dernier est un métal à couche pleine, qui ne présente aucune transition possible en fluorescence. Par contre, avec l'uranium, le tryptophane se désexcite via le métal qui présente des niveaux d'énergie correspondant à une transition fluorescente. L'émission détectée est alors celle du métal (λ>450 nm) et non plus celle du tryptophane. Ce phénomène de transfert d'énergie montre de plus que l'uranium est dans ce complexe situé à moins de 15 Å (distance maximale pour un transfert d'énergie) du tryptophane, ce qui est en accord avec les distances mesurées sur la structure aux rayons X (10 Å et 6,5 Å) entre le tryptophane du peptide et deux des quatre calcium.

Ces résultats indiquent que des protéines caméléons dérivées de la calmoduline, comprenant la séquence d'au moins un peptide selon l'invention peuvent, en présence d'uranium, fixer le peptide substrat MLCK et que peuvent donc être utilisés comme biosenseurs spécifiques de l'uranium VI.

**Exemple 5 : Analyse comparative de l'affinité et de la spécificité des peptides**

**pour l'uranium et le terbium.**

Les affinités comparées pour $UO_2^{2+}$ et $Tb^{3+}$ des peptides dérivés du site I de la calmoduline, mutés au niveaux des résidus tels qu'indiqués dans le tableau III ci-dessous, ont été mesurées en milieu phosphate 1 mM, pH = 7, par la technique, comme décrit à l'exemple 1.
Les résultats sont présentés dans le Tableau III ci-dessous.

**Tableau III: Affinités comparées des peptides pour $UO_2^{2+}$ et $Tb^{3+}$**

| Peptide | Charge | Res20 | Res22 | Res24 | Res31 | Kd($\mu$M) $UO_2^{2+}$ | Kd $Tb^{3+}$ | Kd $Ca^{2+}$ |
|---------|--------|-------|-------|-------|-------|-----------------|--------------|--------------|
| CaM-M1c | 4 | D | D | D | E | 6,8 | 4 $\mu$M | 30 $\mu$M |
| CaM-M2c | 4 | D | D | D | D | > 1000 | 36 $\mu$M | > 10 mM |
| CaM-M3c | 2 | T | D | T | E | 18 | 15 mM | >10mM |
| CaM-M4c | 1 | N | N | N | E | 26 | 12 mM | > 10 mM |
| CaM-M5c | 2 | D | T | T | E | 9,8 | 23 mM | > 10 mM |
| CaM-M6c | 2 | T | T | D | E | 29 | 8,3 mM | > 10 mM |
| CaM-M7c | 2 | S | D | S | E | 53 | 9,3 mM | >10mM |
| CaM-M8c | 1 | T | T | T | E | 54 | 18 mM | > 10 mM |
| CaM-M9c | 3 | T | D | D | E | 15 | 56 $\mu$M | > 10 mM |
| CaM-M10c | 3 | D | D | T | E | 47 | 9,5 mM | > 10 mM |

Ces résultats démontrent que le remplacement dans la boucle complexante de la calmoduline d'un résidu D24 par un résidu neutre, par exemple thréonine, ou de deux résidus des résidus D20, D22 et D24 par deux résidus neutres, par exemple thréonine ou sérine, ou enfin des trois résidus D20, D22 et D24 par des résidus neutres, par exemple thréonine, sérine ou asparagine, induisent une spécificité pour l'uranyle. Pour ces mutants, l'affinité pour les ions calcium ou lanthanides est fortement réduite à la limite de la détection.

**Revendications**

1.  Peptide isolé, **caractérisé en ce qu'**il est sélectionné dans le groupe constitué par les séquences SEQ ID NO : 4 à 7, 9, 10 et 12.

2.  Peptide selon la revendication 1, **caractérisé en ce qu'**il est conjugué à au moins un fluorophore.

3.  Peptide selon la revendication 2, **caractérisé en ce que** ledit fluorophore est une protéine fluorescente sélectionnée parmi : EBFP, ECFP, EYFP, EGFP, DsRed, CopGFP et PhiYFP.

**4.** Peptide selon la revendication 2, **caractérisé en ce que** ledit fluorophore est sélectionné parmi le dansyle, la coumarine, la fluorescéine et les dérivés Alexa.

**5.** Polypeptide, **caractérisé en ce qu'**il comprend la concaténation d'au moins deux peptides identiques ou différents, selon l'une quelconque des revendications 1 à 4.

**6.** Protéine de fusion, **caractérisée en ce qu'**elle est constituée par la fusion de la séquence d'au moins un peptide selon la revendication 1, avec la séquence d'une protéine appropriée.

**7.** Protéine de fusion selon la revendication 6, **caractérisée en ce que** ladite protéine appropriée est sélectionnée dans le groupe constitué par la calmoduline, les protéines caméléons issues de la précédente et les protéines possédant un motif du type hélice-boucle-hélice, capables de lier le calcium.

**8.** Protéine de fusion selon la revendication 6 ou 7, **caractérisée en ce qu'**elle est conjuguée à au moins un fluorophore tel que défini à la revendication 3 ou 4.

**9.** Protéine de fusion selon la revendication 8, **caractérisée en ce que** l'une des extrémités de la dite protéine est couplée à un donneur de fluorescence, et l'autre est couplée à un accepteur de fluorescence.

**10.** Protéine de fusion selon la revendication 9, **caractérisée en ce qu'**elle comprend à l'une de ses extrémités la séquence de l'EBFP ou l'ECFP et à l'autre extrémité la séquence de l'EGFP ou de l'EYFP.

**11.** Molécule d'acide nucléique isolée, **caractérisée en ce qu'**elle comprend une séquence codant pour un peptide, un polypeptide ou une protéine de fusion selon l'une quelconque des revendications 1 à 10.

**12.** Vecteur recombinant eucaryote ou procaryote, **caractérisé en ce qu'**il comprend un insert constitué par une molécule d'acide nucléique selon la revendication 11.

**13.** Cellule eucaryote ou procaryote, **caractérisée en ce qu'**elle est modifiée par un vecteur recombinant selon la revendication 12.

**14.** Organisme animal non-humain transgénique, **caractérisé en ce qu'**il comprend des cellules modifiées par une molécule d'acide nucléique selon la revendication 11.

**15.** Plante transgénique, **caractérisée en ce qu'**elle comprend des cellules modifiées par une molécule d'acide nucléique selon la revendication 11.

**16.** Utilisation d'au moins un peptide isolé selon l'une quelconque des revendications 1 à 4, d'un polypeptide selon la revendication 5 ou d'une protéine de fusion selon l'une quelconque des revendications 6 à 10, pour la préparation d'un réactif destiné à la détection des sols et des eaux contaminés par l'uranium.

**17.** Utilisation d'au moins un peptide isolé selon l'une quelconque des revendications 1 à 4, d'un polypeptide selon la revendication 5 ou d'une protéine de fusion selon l'une quelconque des revendications 6 à 10, pour la préparation d'un réactif destiné au diagnostic d'individus contaminés par l'uranium

**18.** Utilisation d'au moins une cellule selon la revendication 13, pour la remédiation des sols et des eaux contaminés par l'uranium.

**19.** Utilisation d'une plante transgénique selon la revendication 15, pour la remédiation des sols et des eaux contaminés par l'uranium.

**20.** Peptide, polypeptide ou protéine de fusion selon l'une quelconque des revendications 1 à 10 pour utilisation comme médicament pour le traitement des individus contaminés par l'uranium.

**21.** Peptide pour utilisation comme médicament selon la revendication 20, **caractérisé en ce qu'**il est associé à au moins une molécule permettant le ciblage dans le rein et/ou dans les os.

**22.** Peptide pour utilisation comme médicament selon la revendication 20 ou 21, **caractérisé en ce qu'**il est associé à une molécule favorisant son excrétion *in vivo*.

23. Kit pour la détection d'une contamination par l'uranium, **caractérisé en ce qu'**il comprend au moins un peptide isolé, un polypeptide ou une protéine de fusion selon l'une quelconque des revendications 1 à 10.

**Patentansprüche**

1. Isoliertes Peptid, **dadurch gekennzeichnet, dass** es ausgewählt ist aus der Gruppe bestehend aus solchen mit den Sequenzen SEQ ID Nrn. 4 bis 7, 9, 10 und 12.

2. Das Peptid gemäß Anspruch 1, **dadurch gekennzeichnet, dass** es mit mindestens einem Fluorophor konjugiert ist.

3. Das Peptid gemäß Anspruch 2, **dadurch gekennzeichnet, dass** das Fluorophor ein fluoreszierendes Protein ist ausgewählt aus: EBFP, ECFP, EYFP, EGFP, DsRed, CopGFP und PhiYFP.

4. Das Peptid gemäß Anspruch 2, **dadurch gekennzeichnet, dass** das Fluorophor ausgewählt ist aus Dansyl, Cumarin, Fluorescein und Derivate der Alexa-Fluoreszenzfarbstoffe.

5. Polypeptid, **dadurch gekennzeichnet, dass** es die Verknüpfung von mindesétens zwei identischen oder unterschiedlichen Peptiden gemäß irgendeinem der Ansprüche 1 bis 4 enthält.

6. Fusionsprotein, **dadurch gekennzeichnet, dass** es durch die Fusion der Sequenz von mindestens einem Peptid gemäß Anspruch 1 mit einer Sequenz eines entsprechenden Proteins gebildet wird.

7. Das Fusionsprotein gemäß Anspruch 6, **dadurch gekennzeichnet, dass** das entsprechende Protein ausgewählt ist aus der Gruppe bestehend aus Calmodulin, Chamäleon-Proteinen, welche von dem vorhergehenden abgeleitet sind und Proteinen, die ein Helix-Loop-Helix-Motiv aufweisen, die in der Lage sind, Calcium zu binden.

8. Das Fusionsprotein gemäß Anspruch 6 oder 7, **dadurch gekennzeichnet, dass** es mit mindestens einem Fluorophor konjugiert ist, wie dieses in Anspruch 3 oder 4 beschrieben ist.

9. Das Fusionsprotein gemäß Anspruch 8, **dadurch gekennzeichnet, dass** ein Terminus des Proteins mit einem Fluoreszenz-Donor gekoppelt ist und der andere mit einem Fluoreszenzakzeptor gekoppelt ist.

10. Das Fusionsprotein gemäß Anspruch 9, **dadurch gekennzeichnet, dass** es an einem seiner Termini die Sequenz von EBFP oder ECFP und an dem anderen Terminus die Sequenz von EGFP oder EYFP aufweist.

11. Isoliertes Nukleinsäuremolekül, **dadurch gekennzeichnet, dass** es eine Sequenz enthält, welches für ein Peptid, ein Polypeptid oder ein Fusionsprotein gemäß irgendeinem der Ansprüche 1 bis 10 kodiert.

12. Rekombinanter eukaryotischer oder prokaryotischer Vektor, **dadurch gekennzeichnet, dass** er ein Insert enthält bestehend aus einem Nukleinsäuremolekül gemäß Anspruch 11.

13. Eukaryotische oder prokaryotische Zelle, **dadurch gekennzeichnet, dass** sie durch einen rekombinanten Vektor gemäß Anspruch 12 modifiziert ist.

14. Nicht-menschlicher tierischer transgener Organismus, **dadurch gekennzeichnet, dass** er Zellen enthält, die mittels eines Nukleinsäuremoleküls gemäß Anspruch 11 modifiziert sind.

15. Transgene Pflanze, **dadurch gekennzeichnet, dass** sie Zellen enthält, die mittels eines Nukleinsäuremoleküls gemäß Anspruch 11 modifiziert sind.

16. Verwendung von mindestens einem isolierten Peptid gemäß irgendeinem der Ansprüche 1 bis 4, eines Polypeptids gemäß Anspruch 5 oder eines Fusionsproteins gemäß irgendeinem der Ansprüche 6 bis 10, zur Herstellung eines Reagens, welches zum Nachweis von mit Uran kontaminierten Böden und Wasser bestimmt ist.

17. Verwendung von mindestens einem isolierten Peptid gemäß irgendeinem der Ansprüche 1 bis 4, eines Polypeptids gemäß Anspruch 5 oder eines Fusionsproteins gemäß irgendeinem der Ansprüche 6 bis 10, zur Herstellung eines Reagens, welches zum Nachweis von mit Uran kontaminierten Individuen bestimmt ist.

18. Verwendung von mindestens einer Zelle gemäß Anspruch 13 zur Sanierung von mit Uran kontaminierten Böden und Wasser.

19. Verwendung einer transgenen Pflanze gemäß Anspruch 15 zur Sanierung von mit Uran kontaminierten Böden und Wasser.

20. Peptid, Polypeptid oder Fusionsprotein gemäß irgendeinem der Ansprüche 1 bis 10 zur Verwendung als Arzneimittel zur Behandlung von mit Uran kontaminierten Individuen.

21. Das Peptid zur Verwendung als Arzneimittel gemäß Anspruch 20, **dadurch gekennzeichnet, dass** es mit mindestens einem Molekül assoziiert ist, welches die zielgerichtete Anreicherung in den Nieren und/oder in den Knochen erlaubt.

22. Das Peptid zur Verwendung als Arzneimittel gemäß Anspruch 20 oder 21, **dadurch gekennzeichnet, dass** es mit einem Molekül assoziiert ist, welches seine *in vivo* Ausscheidung begünstigt.

23. Kit zum Nachweis einer Kontamination mit Uran, **dadurch gekennzeichnet, dass** es mindestens ein isoliertes Peptid, ein Polypeptid oder ein Fusionsprotein gemäß irgendeinem der Ansprüche 1 bis 10 enthält.

**Claims**

1. An isolated peptide, **characterized in that** it is selected from the group consisting of the sequences SEQ ID NO:4 to 7, 9, 10 to 12.

2. The peptide as claimed in claim 1, **characterized in that** it is conjugated to at least one fluorophore.

3. The peptide as claimed in claim 2, **characterized in that** said fluorophore is a fluorescent protein selected from: EBFP, ECFP, EYFP, EGFP, DsRed, CopGFP and PhiYFP.

4. The peptide as claimed in claim 2, **characterized in that** said fluorophore is selected from dansyl, coumarin, fluorescein and Alexa derivatives.

5. A polypeptide, **characterized in that** it comprises the concatenation of at least two identical or different peptides as claimed in any one of claims 1 to 4.

6. A fusion protein, **characterized in that** it consists of the fusion of at least one peptide as claimed in claim 1, with the sequence of an appropriate protein.

7. The fusion protein as claimed in claim 6, **characterized in that** said appropriate protein is selected from the group consisting of calmodulin, chameleon proteins derived from the latter and proteins having a helix-loop-helix type motif, capable of binding calcium.

8. The fusion protein as claimed in claim 6 or claim 7, **characterized in that** it is conjugated to at least one fluorophore as defined in claim 3 or 4.

9. The fusion protein as claimed in claim 8, **characterized in that** one of the ends of said protein is coupled to a fluorescence donor, and the other is coupled to a fluorescence acceptor.

10. The fusion protein as claimed in claim 9, **characterized in that** it comprises, at one of its ends, the sequence of EBFP or ECFP and, at the other end, the sequence of EGFP or of EYFP.

11. An isolated nucleic acid molecule, **characterized in that** it comprises a sequence encoding a peptide, a polypeptide, or a fusion protein as claimed in any one of claims 1 to 10.

**EP 1 641 815 B1**

12. A eukaryotic or prokaryotic recombinant vector, **characterized in that** it comprises an insert consisting of a nucleic acid molecule as claimed in claim 11.

13. Eukaryotic or prokaryotic cell, **characterized in that** it is modified with a recombinant vector as claimed in claim 12.

14. A transgenic nonhuman animal organism, **characterized in that** it comprises cells modified with a nucleic acid molecule as claimed in claim 11.

15. A transgenic plant, **characterized in that** it comprises cells modified with a nucleic acid molecule as claimed in claim 11.

16. The use of an isolated peptide as claimed in any one of claims 1 to 4, a polypeptide as claimed in claim 5 or a fusion protein as claimed in any one of claims 6 to 10, for preparing a reagent for detecting soils and water contaminated with uranium.

17. The use of an isolated peptide as claimed in any one of claims 1 to 4, a polypeptide as claimed in claim 5 or a fusion protein as claimed in any one of claims 6 to 10, for preparing a reagent for diagnosing individuals contaminated with uranium.

18. The use of at least one modified cell as claimed in claim 13, for the remediation of soils and water contaminated with uranium.

19. The use of a transgenic plant as claimed in claim 15, for the remediation of soils and water contaminated with uranium.

20. Peptide, polypeptide or fusion protein as claimed in any one of claims 1 to 10 for use as a medicament for treating individuals contaminated with uranium.

21. Peptide for use as a medicament according to claim 20, **characterized in that** it is associated with at least one molecule that allows targeting to the kidney and/or to the bone.

22. Peptide for use as a medicament according to claim 20 or 21, **characterized in that** it is associated with a molecule that promotes its excretion *in vivo.*

23. A kit for detecting a contamination with uranium, **characterized in that** it comprises at least: an isolated peptide, a polypeptide or a fusion protein as claimed in any one of claims 1 to 10.

|         | X |   | Y |   | Z | G | # | I | -X |   |   | -Z |
|---------|---|---|---|---|---|---|---|---|----|---|---|----|
| Boucle 1: | D |   | D |   | D |   | T |   | T  |   |   | E  |
| Boucle 2: | D |   | D |   | N |   | T |   | D  |   |   | E  |
| Boucle 3: | D |   | D |   | N |   | Y |   | S  |   |   | E  |
| Boucle 4: | D |   | D |   | N |   | Q |   | N  |   |   | E  |

**Figure 1**

```
position   7                                          39
CaM :      EQIAEFKEAFALFDKDGDGTITTKELGTVMRSL
CaM-M1c :  EQIAEFKEAFALCDKDGDGYITTKELGTCMRSL
CaM-M2c :  EQIAEFKEAFALCDKDGDGYITTKDLGTCMRSL
CaM-M3c:   EQIAEFKEAFALCTKTGDGYITTKELGTCMRSL
CaM-M4c:   EQIAEFKEAFALCNKNGNGYITTKELGTCMRSL
CaM-M5c:   EQIAEFKEAFALCDKTGTGYITTKELGTCMRSL
CaM-M6c :  EQIAEFKEAFALCTKTGDGYITTKELGTCMRSL
CaM-M7c :  EQIAEFKEAFALCSKDGSGYITTKELGTCMRSL
CaM-M8c :  EQIAEFKEAFALCTKTGTGYITTKELGTCMRSL
CaM-M9c    EQIAEFKEAFALCTKDGDGYITTKELGTCMRSL
CaM-M10c   EQIAEFKEAFALCDKDGTGYITTKELGTCMRSL
```

**Figure 2**

FIGURE 3

FIGURE 4

FIGURE 5

FIGURE 6

**Figure 7**

FIGURE 8

FIGURE 9

FIGURE 10

FIGURE 11

FIGURE 12

FIGURE 13

FIGURE 14

FIGURE 15

FIGURE 16

FIGURE 17

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 0308211

**Littérature non-brevet citée dans la description**

- **TSIEN.** Fluorescent chemosensors for ion and molecule recognition. American Chemical Society, 1993, 130-146
- **WALKUP et al.** *J. Am. Soc.,* 1996, vol. 119, 3443-3450
- **GODWIN et al.** *J. Am. Soc.,* 1998, vol. 118, 6514-6515
- **WALKUP et al.** *J. Am. Soc.,* 1997, vol. 119, 3443-3540
- **MIYAWAKI et al.** *Nature,* 1997, vol. 388, 882-887
- **TRUONG et al.** *Nature Struct. Biol.,* 2001, vol. 8, 1069-1073
- **BORIN et al.** *Biopolymer,* 1989, vol. 28, 353-369
- **DADLE et al.** *FEBS Lett.,* 1991, vol. 282, 143, , 146
- **MARSDEN et al.** *Biochem. Cell. Biol.,* 1990, vol. 68, 587-601
- **SHAW et al.** *Science,* 1990, vol. 249, 280-283
- **REID et al.** *Arch. Biochem. Biophys.,* 1995, vol. 323, 115-119
- **PROCYSHYN et al.** *J. Biol. Chem.,* 1994, vol. 269, 1641-1647
- **BECHOR et al.** *Biotechnol.,* 2002, vol. 94, 125-132
- **LEE et al.** *Biosen. Bioelectron.,* 2003, vol. 18, 571-577
- **LE CLAINCHE et al.** *J. Biol. Inorg. Chem.,* 2003, vol. 8, 334-340
- **BOWEN B. et al.** *Photochem. Photobiol.,* 2003, vol. 77 (4), 362-369
- **FREDERICK M. AUSUBEL.** Current Protocols in Molecular Biology. Wiley and son Inc, 2000
- **WHITTAL et al.** *Prot. Sci.,* 2000, vol. 9, 332-343
- **LINSE et al.** *J. Biol. Chem.,* 1991, vol. 266, 8050-8054
- **KUZMIC, P.** *Anal. Biochem.,* 1996, vol. 237, 260-273

SEQUENCE LISTING

<110> COMMISSARIAT A L'ENERGIE ATOMIQUE (CEA) VITA, Claudio LE CLAINCHE, Loïc MONJARDET, Véronique

<120> PEPTIDES CHELATANT L'URANIUM ET LEURS APPLICATIONS

<130> F263PCT89

<150> FR 0308211
<151> 2003-07-04

<160> 12

<170> PatentIn version 3.1

<210> 1
<211> 33
<212> PRT
<213> Artificial sequence

<220>
<223> peptide CaM

<400> 1

Glu Gln Ile Ala Glu Phe Lys Glu Ala Phe Ala Leu Phe Asp Lys Asp
1                   5                   10                  15

Gly Asp Gly Thr Ile Thr Thr Lys Glu Leu Gly Thr Val Met Arg Ser
              20                  25                  30

Leu

<210> 2
<211> 33
<212> PRT
<213> Artificial sequence

<220>
<223> peptide CaM-M1c

<400> 2

Glu Gln Ile Ala Glu Phe Lys Glu Ala Phe Ala Leu Cys Asp Lys Asp
1                   5                   10                  15

Gly Asp Gly Tyr Ile Thr Thr Lys Glu Leu Gly Thr Cys Met Arg Ser
              20                  25                  30

Leu

<210> 3
<211> 33
<212> PRT
<213> Artificial sequence

<220>
<223> peptide CaM-M2c

<400> 3

Glu Gln Ile Ala Glu Phe Lys Glu Ala Phe Ala Leu Cys Asp Lys Asp
1                   5                   10                  15

Gly Asp Gly Tyr Ile Thr Thr Lys Asp Leu Gly Thr Cys Met Arg Ser
              20                  25                  30

Leu

<210> 4
<211> 33
<212> PRT
<213> Artificial sequence

<220>
<223> peptide CaM-M3c

<400> 4

```
        Glu Gln Ile Ala Glu Phe Lys Glu Ala Phe Ala Leu Cys Thr Lys Asp
        1               5                   10                  15

        Gly Thr Gly Tyr Ile Thr Thr Lys Glu Leu Gly Thr Cys Met Arg Ser
                    20                  25                  30

        Leu
```

<210> 5
<211> 33
<212> PRT
<213> Artificial sequence

<220>
<223> peptide CaM-M4c

<400> 5

```
        Glu Gln Ile Ala Glu Phe Lys Glu Ala Phe Ala Leu Cys Asn Lys Asn
        1               5                   10                  15

        Gly Asn Gly Tyr Ile Thr Thr Lys Glu Leu Gly Thr Cys Met Arg Ser
                    20                  25                  30

        Leu
```

<210> 6
<211> 33
<212> PRT
<213> Artificial sequence

<220>
<223> peptide CaM-M5c

<400> 6

```
        Glu Gln Ile Ala Glu Phe Lys Glu Ala Phe Ala Leu Cys Asp Lys Thr
        1               5                   10                  15

        Gly Thr Gly Tyr Ile Thr Thr Lys Glu Leu Gly Thr Cys Met Arg Ser
                    20                  25                  30

        Leu
```

<210> 7
<211> 33
<212> PRT
<213> Artificial sequence

<220>
<223> peptide CaM-M6c

<400> 7

```
Glu Gln Ile Ala Glu Phe Lys Glu Ala Phe Ala Leu Cys Thr Lys Thr
1               5               10              15

Gly Asp Gly Tyr Ile Thr Thr Lys Glu Leu Gly Thr Cys Met Arg Ser
            20              25              30

Leu
```

<210> 8
<211> 17
<212> PRT
<213> Artificial sequence

<220>
<223> peptide MLCKp

<400> 8

```
Arg Arg Lys Trp Gln Lys Thr Gly His Ala Val Arg Ala Ile Gly Arg
1               5               10              15

Leu
```

<210> 9
<211> 33
<212> PRT
<213> Artificial sequence

<220>
<223> peptide CaM-M7c

<400> 9

```
Glu Gln Ile Ala Glu Phe Lys Glu Ala Phe Ala Leu Cys Ser Lys Asp
1               5               10              15

Gly Ser Gly Tyr Ile Thr Thr Lys Glu Leu Gly Thr Cys Met Arg Ser
            20              25              30

Leu
```

<210> 10
<211> 33
<212> PRT
<213> Artificial sequence

<220>
<223> peptide CaM-M8c

<400> 10

```
Glu Gln Ile Ala Glu Phe Lys Glu Ala Phe Ala Leu Cys Thr Lys Thr
1               5               10              15

Gly Thr Gly Tyr Ile Thr Thr Lys Glu Leu Gly Thr Cys Met Arg Ser
            20              25              30

Leu
```

<210> 11
<211> 33
<212> PRT
<213> Artificial sequence

<220>
<223> peptide caM-M9c

<400> 11

```
Glu Gln Ile Ala Glu Phe Lys Glu Ala Phe Ala Leu Cys Thr Lys Asp
1               5               10              15

Gly Asp Gly Tyr Ile Thr Thr Lys Glu Leu Gly Thr Cys Met Arg Ser
            20              25              30

Leu
```

<210> 12
<211> 33
<212> PRT
<213> Artificial sequence

<220>
<223> peptide CaM-M10c

<400> 12

```
Glu Gln Ile Ala Glu Phe Lys Glu Ala Phe Ala Leu Cys Asp Lys Asp
1               5               10              15

Gly Thr Gly Tyr Ile Thr Thr Lys Glu Leu Gly Thr Cys Met Arg Ser
            20              25              30

Leu
```